# EUROPEAN PATENT APPLICATION

(11) **EP 1 741 720 A1**
(43) Date of publication of application: **10.01.2007**
(21) Application number: 06119421.3
(22) Date of filing: 11.07.2002
(51) Int. Cl.: C07K 14/30, C07K 14/055

(54) **Modified DNA molecule, recombinant containing the same, and uses thereof**

(30) Priority: 11.07.2001 US 901572; 25.04.2002 US 131591 P
(62) Divisional of application: 02254879.6
(71) Applicant: ZEON CORPORATION, Chiyoda-ku Tokyo 100-8323 (JP)
(72) Inventor: Okuda, Takashi Zeon Corp. Res. & Devel. Center, Kawasaki-shi (JP); Saito, Shuji Zeon Corp. Res. & Devel. Center, Kawasaki-shi (JP); Dorsey, Kristi M., Lenexa, KS 66215 (US); Tsuzaki, Yoshinari, Lenexa, KS 66215 (US)
(74) Representative: Jones, Helen M.M.

(57) **Abstract**

There is provided a DNA molecule derived from a prokaryotic cell in which at least one of the DNA regions encoding NXB (N is asparagine, X is any amino acid other than proline, and B is serine or threonine) has been modified so that no N-glycosylation occurs during the expression in a eukaryotic cell, and since the DNA molecule has been modified at the N-glycosylation site, it produces a non-N-glycosylated protein, which thereby exhibits a high immunogenicity when, for example, it is allowed to produce, in a eukaryotic cell, an antigen protein derived from a prokaryotic cell.

## Description

### Technical Field

The present invention relates to an modified gene derived from a prokaryotic cell (including an organism comprising prokaryotic cells) said gene capable of producing a protein that has no sugar chain additions when expressed in a eukaryotic cell (including an organism comprising eukaryotic cells), and uses thereof.

### Background

As methods of obtaining gene products of prokaryotic cells such as bacteria and blue-green algae, there have conventionally been used methods of culturing prokaryotic cells having said gene and then isolating and purifying the gene products of interest. However, when the gene product thus obtained is used as a vaccine, such methods had a problem of production efficiency in which an adequate amount of expression cannot be secured, and a safety problem due to difficulty in removing impurities such as pyrogens in the purification process.

Thus, focusing on the advantage of obviating the need of removing pyrogens, attempts have been made to introduce into a eukaryotic cell a recombinant vector in which the gene of interest derived from a prokaryotic cell has been integrated into a vector such as a virus, and then allowing the gene to be directly expressed in the eukaryotic cell. However, since prokaryotic cells and eukaryotic cells are essentially different in their mode of gene expression, gene products of a prokaryotic cell expressed in a eukaryotic cell could not exhibit the activity at a level equivalent to those produced in the prokaryotic cell, which sometimes resulted in an inadequate immunogenicity.

For example, U.S. Pat. No. 5,871,742 describes that an avipoxvirus vector which has integrated an antigen gene TTM-1 (TTM-1 gene) derived from Mycoplasma gallisepticum is effective as a vaccine to protect against Mycoplasma gallisepticum infection. Subsequently, it was found that the product (TTMG-1 antigen) of the TTM-1 gene is displayed on the cell membrane when the TTM-1 gene is expressed in the prokaryotic cell, whereas the TTM-1 product expressed in a eukaryotic cell is not displayed on the cell membrane of the eukaryotic cell and thereby is unlikely to exhibit the inherent immunogenicity. As a result of further study, in order to display the TTM-1 product on the surface of eukaryotic cells, a fusion gene was constructed in which a DNA encoding a virus-derived type II signal sequence such as signal sequence (hereinafter referred to as MDV gB signal) of gB of Marek's disease virus (MDV) has been ligated to said gene. By integrating this fusion gene into avipoxvirus and allowing it to be expressed, the TTMG-1 antigen was successfully displayed on the surface of the cell membrane, and thereby a vaccine that exhibits a higher activity of protecting against infection was obtained (International Patent Publication WO97/36924).

In most cases, proteins synthesized in the eukaryotic cell are different from those synthesized in the prokaryotic cell in that the former has sugar chains attached thereto.

Yoshida et al. (2000) constructed a recombinant avipoxvirus in which mgc3 (the mgc3 gene), a gene derived from Mycoplasma gallisepticum, other than the TTM-1 gene was integrated, and investigated the expression of the product (the MGC3 antigen) of the mgc 3 gene by immunoprecipitation. As a result, it was confirmed that N-linked sugar chains are not attached (N-glycosylated) to the MGC3 antigen having no MDVgB signal added thereto whereas the MDVgB signal-added MGC3 antigen undergoes N-glycosylation. Yoshida et al. (2000) also confirmed that the MGC3 fusion protein produced by a recombinant avipoxvirus that has integrated therein a fusion gene of the mgc3 gene and DNA encoding the MDVgB signal is 50-fold more reactive to a monoclonal antibody 35A6 that recognizes the MGC3 protein than the MGC3 antigen produced by a recombinant avipoxvirus that has integrated therein only the antigen gene mgc3. Based on this, it had been thought that though the fusion of a DNA encoding the MDVgB signal with the antigen gene may result in the addition of sugars to the protein obtained, a highly immunogenic protein could be obtained without N-glycosylation affecting immunogenicity.

### Disclosure of Invention

However, the inventors of the present invention have performed an animal experiment in which chickens were inoculated with a recombinant virus prepared according to Yoshida et al. (2000) by integrating the antigen gene of Mycoplasma gallisepticum to which a DNA encoding the MDVgB signal had been added, and thereby confirmed that no significant enhancement in the effect of protecting against infection was observed compared to when the DNA encoding the MDVgB signal was not added.

This result demonstrated that the use of a fusion gene to which a signal-encoding DNA is added does not always produce enhanced immunogenicity.

Accordingly, after intensive study in order to obtain a novel vaccine having an enhanced immunogenicity, the inventors of the present invention have found that the antigen protein that should inherently be produced by the prokaryote, i.e. an antigen protein that is not N-glycosylated, provides a high immunogenicity, and thereby have completed the present invention.

Thus, according to the present invention:
Firstly, there is provided a DNA molecule derived from a prokaryotic cell in which at least one of the DNA regions encoding NXB (N is asparagine, X is any amino acid other than proline, and B is serine or threonine) has been modified so that no N-glycosylation occurs during the expression in the eukaryotic cell;
secondly, there is provided a fused DNA molecule in which a DNA encoding a signal sequence is ligated to the N-terminal end of said modified DNA molecule so that it may be expressed as a fusion protein;
thirdly, there is provided a recombinant virus that has integrated therein (1) a DNA molecule in which at least one of the DNA regions encoding NXB (N is asparagine, X is any amino acid other than proline, and B is serine or threonine) has been modified so that no N-glycosylation occurs during the expression in the eukaryotic cell, or (2) a fused DNA molecule in which a DNA encoding a signal sequence is ligated to the N-terminal end of said modified DNA molecule so that it may be expressed as a fusion protein;
fourthly, there is provided a method of producing a protein encoded by said modified DNA molecule or said fused DNA molecule using said recombinant virus in a eukaryotic cell; and
fifthly, there is provided a vaccine that uses said recombinant virus.

### Brief Explanation of Drawings

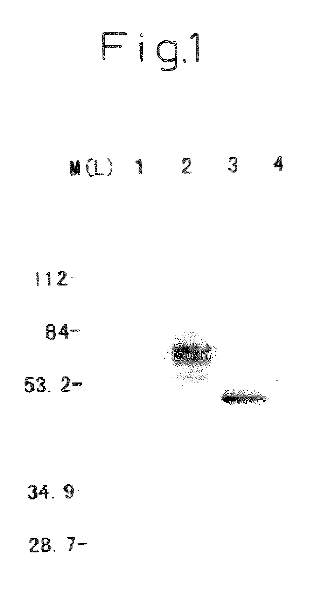

Figure 1 is a Western blot that compares the molecular weight detected using anti-TTMG-1 antiserum of the non-N-glycosylated TTMG-1 antigen (lane 3) expressed from a recombinant virus containing the modified DNA and the N-glycosylated TTMG-1 antigen (lane 2) expressed from a recombinant virus containing the unmodified DNA. In this figure, lane 1 indicates the result from the virus (negative control) containing no antigen gene and lane 4 indicates the result of the MGC3 antigen that cannot be detected with the anti-TTMG-1 antiserum.

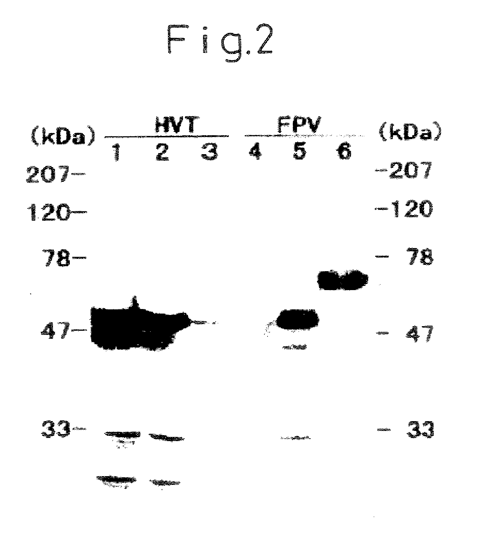

Figure 2 is a Western blot that compares the molecular weight detected using anti-TTMG-1 antiserum of the non-N-glycosylated TTMG-1 fusion protein (lane 5) expressed from the FPV containing the fused DNA of a DNA encoding the modified TTMG-1 antigen and a DNA encoding the signal sequence MDVgB, the non-N-glycosylated TTMG-1 fusion protein (lane 1 and 2) expressed from the HVT containing said fused DNA, and the N-glycosylated TTMG-1 fusion protein (lane 6) expressed from the FPV containing the unmodified DNA. In this figure, lane 4 indicates the result from FPV (negative control) containing no antigen gene and lane 3 indicates the result of the antigen protein NDV that cannot be detected with the above antiserum.

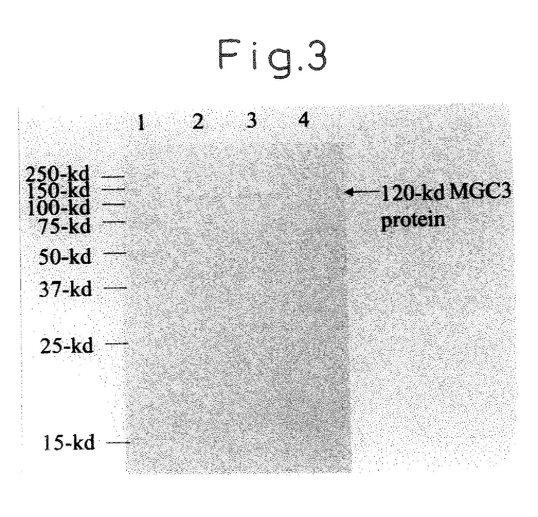

Figure 3 is a Western blot that shows that the molecular weight of the non-N-glycosylated fusion protein expressed from the FPV containing the fusion of a DNA encoding the modified MGC3 antigen and a DNA encoding the signal sequence MDVgB is 120 kd. It has been separately confirmed that the molecular weight of the N-glycosylated MGC3 antigen expressed from the unmodified mgc3 gene is 140 kd. In this figure, lane 1 indicates the molecular weight standard and lane 4 indicates the result from the virus containing no antigen gene.

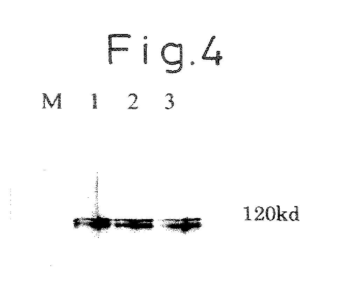

Figure 4 is a Western blot that shows that the treatment of the non-N-glycosylated MGC3 antigen (molecular weight 120 kd) expressed from the virus into which an modified mgc3 gene was introduced with a sugar chain-cleaving enzyme endoglycosidase or PNGase F does not change (reduce) the molecular weight.

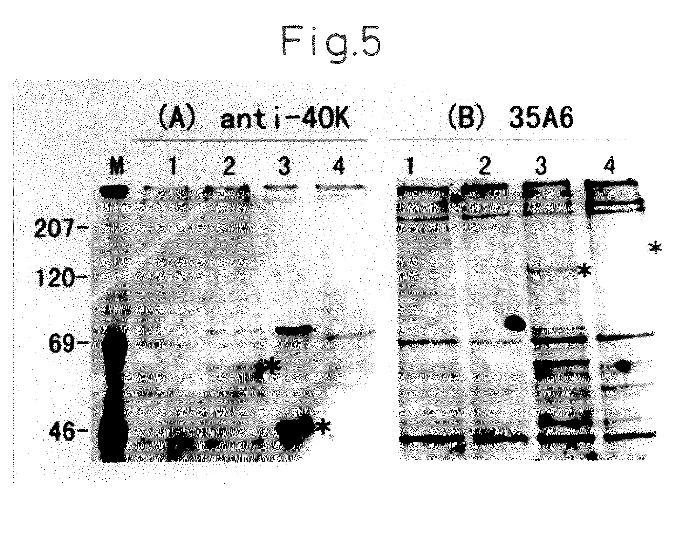

In Figure 5, A indicates the result in which the antigen protein was detected by immunoprecipitation with anti-TTMG-1 (anti-40K) antiserum, and B indicates the result in which it was detected by immunoprecipitation with the monoclonal antibody 35A6 that reacts with the antigen protein MGC3. In both of panel A and B, lane 1 indicates the result from the virus (parent FPV) containing no antigen gene, lane 2 indicates the result from the virus containing the unmodified TTMG-1 gene that expresses the glycosylated TTMG-1 antigen, lane 3 indicates the result from the virus containing the modified TTMG-1 gene that expresses the non-N-glycosylated TTMG-1 antigen and the modified mgc3 antigen gene that expresses the non-N-glycosylated MGC3 antigen, and lane 4 indicates the result from the virus containing the unmodified mgc3 gene that expresses the N-glycosylated MGC3 antigen.

### Best Mode for Carrying Out the Invention

In accordance with the present invention, the amino acid sequence represented by NXB (N is asparagine, X is any amino acid other than proline, and B is serine or threonine) is an amino acid that is present in a peptide encoded by a DNA molecule derived from a prokaryotic cell and that is recognized as a N-glycosylation site in a eukaryotic cell. According to the present invention, this amino acid sequence is sometimes referred to as "a potential N-glycosylation site".

Known as such are an amino acid sequence represented by N (asparagine)-X (X is any amino acid other than proline)-S (serine), or an amino acid sequence represented by N (asparagine)-X (X is any amino acid other than proline)-T (threonine).

In accordance with the present invention, the DNA region that is modified so as not to be N-glycosylated (the addition of a N-linked sugar chain) is a DNA region that encodes one or more N-glycosylation sites present in the gene derived from a prokaryotic cell. When a DNA region encoding a plurality of N-glycosylation sites is present in the gene of a prokaryotic cell, the DNA region encoding the N-glycosylation site that is displayed on the surface may be only modified considering the conformation of the protein finally obtained, or all such regions present in said gene may be modified.

### Modified DNA molecule

The modified DNA molecule of the present invention is a DNA molecule in which at least one region among the DNA regions encoding potential N-glycosylation sites present in the gene derived from a prokaryotic cell has been modified so that N-glycosylation does not occur during the expression in a eukaryotic cell for.

### (Prokaryotic cell)

According to the present invention, prokaryotic cells may be bacteria or blue green algae, and pathogenic bacteria are suitable for the purpose of the present invention. For example, there can be mentioned gram-positive bacteria such as Staphylococcus aureus, Clostridium tetani, and Clostridium botulinum; gram-negative bacteria such as Escherichia coli, Salmonella, Haemophilus, and Bordetella; acid-fast bacteria such as Mycobacterium tuberculosis; and Mycoplasma, and preferably there can be mentioned Mycoplasma having pathogenically against avians.

### (The prokaryotic cell-derived gene to be the target of alteration)

According to the present invention, the prokaryotic cell-derived gene to be the target of alteration is a portion of the gene that is owned by the above prokaryotic cell and that contains the gene region encoding gene products such as protein. When it is used as vaccine specifically, the gene preferably contains a portion having all or one or more epitopes of the antigen gene that encodes the antigen protein.

As the antigen gene, there can be mentioned adhesin present in various bacteria, and more preferably genes encoding Pertactin, Fimbriae, Filamentous hemagglutinin (FHA) from Bordetella, the TTM-1 gene as set forth in SEQ ID NO: 1 that encodes the TMG-1 antigen (29 kd) described in U.S. Pat. No. 5,766,594, the TTMG-1 antigen (40 kd) described in U.S. Pat. No. 5,489,430, and the TM-66 antigen (66 kd) or TM-67 antigen (67 kd) described in U.S. Pat. No. 5,871,742, and the mgc3 gene as set forth in SEQ ID NO: 2 (GeneBank accession No. AB023292) encoding the M11 antigen (this is identical with the MGC3 antigen used by Yoshida et al.) described in International Patent Publication WO97/24370.

By analyzing the base sequence of the prokaryotic cell-derived gene to be the target of alteration and estimating the amino acid sequence from the sequence, it is possible to specify the DNA region that encodes the potential N-glycosylation site in said gene.

When a translation codon inherent to the prokaryotic cell is present in said gene used, this portion can be modified as needed. In the case of genes derived from Mycoplasma, for example, the base sequence "TGA" that should be read as the terminal codon in the usual prokaryotic cell is translated as tryptophan, and thus this "TGA" can be modified so that it may be translated as an amino acid (when TGA is to be translated as tryptophan, it is changed to TGG) in the eukaryotic cell.

Furthermore, when poxvirus is used to express protein, the base sequence "T5NT" is likely to become a translational termination signal (Yanagida et al., 1992), it is preferred to alter so that it is not accompanied by amino acid substitution in order to prevent the termination of translation.

In addition, as reported that after the Gp120 gene of HIV was converted to a human optimized codon, its effect as a DNA vaccine increased (Andre et al., 1998), the amount expressed of a gene can be enhanced by replacing with a translation codon (optimization of codon) of an amino acid that is highly frequently used in the living body in which the DNA molecule is to be expressed. Accordingly, when the DNA molecule of the present invention is to be expressed in chickens, it is likely that optimization to a chicken codon can enhance the gene expression, and the alteration to a chicken codon can be performed as needed.

### (Method of altering the N-glycosylation site)

As the method of altering the N-glycosylation site in the prokaryotic cell-derived gene, there can be mentioned the following three alteration methods:
(1) the alteration of the DNA sequence encoding asparagine (N) to a DNA sequence encoding an amino acid other than asparagine;
(2) the alteration of the DNA sequence encoding any amino acid (X) other than proline to a DNA sequence encoding proline; and
(3) the alteration of the DNA sequence encoding serine or threonine (B) to a DNA sequence encoding an amino acid other than serine or threonine.

Among these three methods, the method of (1) in which the DNA sequence encoding asparagine (N) is changed to a DNA sequence encoding glutamine (Q) is preferred in that it does not affect protein conformation.

These methods of altering genes are not specifically limited. For example, methods using synthetic DNA such as in vitro mutagenesis and polymerase chain reaction (PCR) are commonly used. From the viewpoint that a plurality of mutations can introduced and in a shorter time as well, the method of using PCR is preferred.

### Fused DNA molecule

In the fused DNA molecule of the present invention, a DNA encoding a signal sequence has been ligated to the N-terminal end of the above DNA molecule of the present invention so that it may be expressed as a fusion protein.

The ligation of a DNA encoding a signal sequence to the N-terminal end of the DNA molecule of the present invention followed by the expression of the fusion protein makes it possible to obtain a vaccine having a higher immunogenicity.

### (Signal sequence and DNA encoding the same)

Signal sequences and methods of ligating the DNA encoding the same to another DNA molecule have been described in detail in U.S. Pat. No. 5,871,742, International Patent Publication WO97/36924, and Yoshida et al. (2000), and are briefly explained hereinbelow.

Signal sequences are sequences that function when membrane proteins of viruses etc. are extracellularly secreted or displayed. The membrane protein from which signal sequences are derived may be either type I or type II. Examples of membrane proteins include the hemagglutinin neuraminidase (HN) protein of Newcastle disease virus NDV), the gB protein of herpes simplex virus (HSV) type 1 and Marek's disease virus (MDV) type I, the G glycoprotein of Rabies virus, and the like. Signal sequences can be readily found by analyzing the amino acid sequence of the hydrophobic peptide region in the amino terminal or the carboxy terminal of the membrane protein.

DNA encoding the signal sequence thus specified may be obtained by a conventional method, and ligated to the DNA molecule of the present invention to obtain the fused DNA molecule of the present invention. At this time, in order to allow the expression as a fusion protein, the signal sequence is ligated to the N-terminal end of the DNA molecule using a conventional method in such a manner that the open reading frame (ORF) estimated by analyzing the base sequence of the DNA molecule becomes aligned in frame.

When a potential N-glycosylation site is present in such a signal sequence, it is preferred to alter the DNA encoding the signal sequence as for the DNA molecule of the present invention in a similar manner as described above so that it may be not recognized as a N-glycosylation site when expressed in a eukaryotic cell.

### Recombinant virus

The recombinant virus of the present invention has integrated in the genome thereof the DNA molecule or the fused DNA molecule (hereinafter referred to collectively as the "DNA molecule" of the present invention).

When the DNA molecule of the present invention is to be integrated in the recombinant virus, it is usually integrated to be placed under the control of a regulatory gene (promoter) in order to obtain a high amount of expression. The promoter may be commonly used one that functions in the eukaryotic cell, and may be derived from a eukaryotic cell or virus. Specific examples of promoters include thymidine kinase promoter of herpesvirus (Ross et al., 1993), the gB protein promoter of the herpesvirus of turkey (HVT) and Marek's disease virus (MDV) serotype I (Ross et al., 1993), the IE promoter of human cytomegalovirus (HCMV) (Stinski et al., 1995), SV40 promoter (Gunning et al., 1987), human β actin promoter (Gunning et al., 1987), chicken β actin promoter (Kost et al., 1983), the LTR promoter of Rous sarcoma virus (RSV) (Greuel et al., 1990), chimera promoter (Japanese Unexamined Patent Publication (Kokai) No. 2001-188), and the like.

By adding an enhancer, a factor that activates transcription, in addition to promoter, a more efficient expression may be predicted (Stinski et al., 1995). As the enhancer, there can be mentioned a portion of the cytomegalovirus-derived promoter, and generally the positional relationship to the inserted gene is not limited.

Furthermore, in the case of a recombinant herpesvirus, a higher amount of expression is obtained by adding DNA encoding a polyadenylation signal to the downstream of the DNA molecule of the present invention. As the polyadenylation signal, there can be illustrated the Poly A signal (Gunning et al., 1987) of SV40 etc. and the Poly A signal (Yanagida et al., 1993) of UL46h, UL47h and UL49h of Marek's disease virus (MDV) serotype I.

The method of obtaining the recombinant virus of the present invention is not specifically limited, and for example there is the method of homologous recombination between a vector in which any DNA (the DNA inserted into virus is hereinafter referred to collectively as foreign gene) such as the DNA molecule of the present invention and a promoter was sandwiched in between the fragments of the gene region that is not essential for the viral replication.

The recombinant virus of the present invention will now be explained in detail with reference to the example of a recombinant herpesvirus and a recombinant poxvirus.

### (Recombinant virus)

As the gene region nonessential for the growth of herpesvirus in the case of Marek's disease virus (MDV) serotype 1, serotype II, serotype III (type III is turkey herpesvirus), there can be illustrated the TK region (Ross L. et al., 1991), US10 region (Sakaguchi M. et al., 1994), the US2 region (Sondermeijer, P.j. et al., 1993), the region in between UL44 and UL45 and the region in between UL45 and UL46 (SEQ ID NO: 3 indicates a sequence from UL44 to UL46) described in International Patent Publication WO99/18215.

A transfer vector for recombination is constructed that has a DNA region in which necessary foreign genes such as the DNA of the present invention and promoters are sandwiched by the nonessential region. The length of the region into which foreign genes such as the DNA of the present invention are inserted is, but not limited to, about 10 bp each in front of and behind the foreign gene insertion site, preferably 100 bp or longer, more preferably 500 bp or longer. The vector may be one that is generally used for the construction of recombinant virus, and include, for example, plasmid such as pBR322, pBR325, pBR327, pBR328, puC8, pUC18, and pUC19, and phage such as λ phage and M13 phage, and cosmid such as pHC9.

Homologous recombination between this recombinant vector and the parent herpesvirus is allowed to take place to construct a recombinant herpesvirus.

The parent herpesvirus may be any herpesvirus that infects mammals or avians. In order to obtain avian vaccines, Marek's disease virus is preferably selected. There are three types of Marek's disease virus: serotype I, II, and III, any of which may be used to obtain the recombinant herpesvirus of the present invention. These Marek's disease viruses may be naturally occurring ones or may be those available from ATCC etc. with or without charge, and most preferably they are non-phathogenic. As such viruses, there can be illustrated the CVI988 (Rispens) strain for Marek's disease virus serotype I, the SB-1 strain for Marek's disease virus serotype II, and FC126 (ATCC VR-584B), PB-THV1, H-2, YT-7, and HPRS-26 for Marek's disease virus serotype III (HVT).

Specific examples for generating recombinant herpesvirus include those described below.

A recombinant vector in which the above-mentioned foreign gene has been sandwiched in a nonessential insertion site is introduced into a herpesvirus-infected cell by electroporation, the calcium phosphate method, a method that uses lipofectin, the gene gun method, and the like. In the case of avian herpesviruses, for example, cells to which herpesvirus is infected are preferably derived from avians, and include, for example, chick embryo fibroblasts (CEFs), developing chicken embryos, and chicken kidney cells. Culturing of infected cells may be performed by a commonly used method. As the method of introducing a recombinant vector into infected cells, electroporation and a method that employs lipofectin are preferably adopted because of their high efficiency of introduction. When the amount of plasmid introduced is in the range of 0.1 to 1000 µg, the incidence of recombinant virus between the genomic DNA of herpesvirus and the homologous region of the recombinant vector becomes higher. In order to select only recombinant viruses that have introduced such a plasmid, the Black Plaque Assay (BPA) can be used. The BPA method is one in which the foreign gene product and an antibody against this are reacted, to which an enzyme-labeled second antibody is reacted, and then the substrate for said enzyme is used to visualize plaques that expressed the products of the foreign gene. Using this method, a recombinant virus that expressed the foreign antigen gene may be selected. When these recombinant viruses are constructed, due to ease of detection, a marker gene such as β-galactosidase may be integrated as one of the foreign genes, in which expression is easily monitored using Bluo-Gal (manufactured by Gibco BRL) to isolate a recombinant. In addition, the method of plaque hybridization etc. may be used to isolate the recombinant herpesvirus of interest. By repeating these procedures, recombinant virus is purified.

### (Recombinant poxvirus)

Recombinant poxvirus can be constructed, in a similar manner to the construction of recombinant herpesvirus, using homologous recombination.

However, since poxvirus per se has a transcription factor etc. and thus transcription and translation are likely to occur by constituents of the virus per se. Therefore, the promoter used is not the one mentioned above as usable for the construction of the above recombinant herpesvirus but must be changed to a promoter for poxvirus. As the promoter for poxvirus, a synthetic promoter that functions as a promoter in the virus belonging to the poxvirus with reference to Davison et al. 1 and 2 (1989), or a natural promoter of poxvirus may be used. As the natural promoter of poxvirus, there can be mentioned the 7.5K promoter, 19K polypeptide promoter, 42K polypeptide promoter, 28K polypeptide promoter, and TK promoter (Weir et al., 1984) of vaccinia virus.

When a recombinant poxvirus is used for the expression of proteins, as described above, the foreign gene used is preferably modified at the T5NT region.

As the gene region nonessential for the growth of poxvirus, there can be used the TK gene region of fowlpox virus, pigeon poxvirus, quail pox, turkey poxvirus etc., the region in between the open reading frames (ORF) as described in U.S. Pat. No. 5,180,675 and Japanese Patent Publication No. 2766984, and the region described in U.S. Pat. No. 5,387,519. More specifically, an EcoRI fragment (7.3 kbp), an EcoRI-HindIII fragment (about 5.0 kbp), a BamHI fragment (about 4.0 kbp), and a HindIII fragment (about 5.2 kbp) derived from pigeon pox described in U.S. Pat. No. 5,387,519, and SpeI-HpaI (3026 bp) (SEQ ID NO: 4) in an EcoRI fragment (7.3 kbp).

### Production of proteins

By infecting the recombinant virus of the present invention to the cells in which said virus can infect and propagate, and culturing the cells, the DNA molecule derived from a prokaryotic cell integrated into the recombinant virus may be expressed and the protein encoded by said DNA molecule can be obtained.

The cells to which the virus is infected used may be the same cells as those used in the construction of the recombinant virus mentioned above. The culture conditions are the same as well.

The method of purifying the protein obtained is not specifically limited, and may be isolated and purified according to the description in, for example, Methods in Enzymology, Vol. 182 ("Guide to Protein Purification", Murry P. Desutscher ed., published by Academic Press).

The antigen peptide thus obtained may be diluted according to a conventional method, or mixed with a suitable adjuvant, and may be used as a component vaccine. As the adjuvant used, there can be illustrated Freund's complete adjuvant, Freund's incomplete adjuvant, alum, Arlacel, and the like. The mixed ratio with the adjuvant is, but not limited to, generally 1:1. When used as a component vaccine, 0.1 µg or more per individual, for chicken for example, may be administered, and there is no specific upper limit unless acute toxicity manifests. The method of administration may be subcutaneous, intravenous, intramuscular, or intraperitoneal injection, a method of immunizing by spraying into the airway, administration by drinking water and the like.

### Vaccine

### (Recombinant herpesvirus vaccine)

The method of preparing live vaccine comprising a recombinant herpesvirus as a main ingredient is not specifically limited. For example, the following method may be used for preparation.

The recombinant herpesvirus of the present invention is infected to the cells (hereinafter referred to as host cells) in which said virus can grow, and after the cells have grown, they are scraped using a scraper or trypsin, and then centrifuged to separate the supernatant from the infected cells. In the case of avian herpesvirus, for example, the host cell used is preferably one derived from avians and CEF, chicken kidney cells etc. are preferably used. The infected cells thus obtained are suspended into a culture medium containing 10% dimethyl sulfoxide (DMSO), and stored in the presence of liquid nitrogen. When used as vaccine, the frozen product is thawed and dissolved in 100 volumes of phosphate buffer or physiological saline and used.

Stabilizers and other components for storing the above infected cells under liquid nitrogen are not specifically limited as long as they allow stable survival of the virus-infected cells and are components that do not pose any pharmacological problems to recipients.

The administration method of the recombinant herpesvirus thus prepared is not specifically limited. For example, there can be mentioned methods similar to those currently used for herpesvirus vaccines such as a method of subcutaneously injecting to the chicken individual, and a method of inoculating by making a hole during the development of the developing chicken embryos.

The amount and the timing of inoculation may the same as the conventional vaccines. For example, by inoculating subcutaneously 10²-10⁴ PFU or 10²-10⁴ TCID₅₀ to the dorsum of the chicken on the day of hatching using a 26G needle, the effect as vaccine can be expected. The recombinant herpesvirus prepared as described above functions as a vaccine against not only the pathogenic prokaryotic organisms from which the DNA molecule of the present invention was obtained but also the parent herpesvirus.

### (Recombinant poxvirus vaccine)

In a completely similar manner to the recombinant herpesvirus etc. mentioned above, the recombinant poxvirus can be prepared as vaccine using a similar procedure. However, unlike Marek's disease virus serotype I, II, and III described as examples of recombinant herpesvirus, it is not necessary to suspend the infected cells into a culture medium containing 10% dimethyl sulfoxide (DMSO) and to store them frozen under liquid nitrogen, but the supernatant containing the recombinant poxvirus can be obtained by harvesting and homogenizing the infected cells followed by centrifugation and the like. The supernatant is generally stored lyophilized, and, as appropriate, mixed with a pharmaceutically acceptable carrier or physiological saline and then used as a vaccine, but it is possible to add a carrier or physiological saline without lyophilization and to use as a vaccine.

The method of inoculating the recombinant poxvirus, the dosage and the timing of inoculation may be the same as conventional vaccines. For example, in the case of recombinant avipoxvirus vaccine, 10²-10⁴ PFu or 10²-10⁴ TCID₅₀ is stabbed to the wing web of a chicken one week after hatching using a puncture needle.

The recombinant poxvirus thus prepared functions as a vaccine against not only the pathogenic prokaryotic organisms from which the DNA molecule of the present invention was obtained but also the parent poxvirus.

Furthermore, the recombinant vectors constructed in the construction of the recombinant virus of the present invention can be used per se as a DNA vaccine. This is a method of expressing an antigen by inoculating the purified vector directly into the eukaryotic cell individual. It can be injected or inoculated by making scratches alone or together with those that supplement expression or immunological ability, or can be introduced into a eukaryotic cell individual by the gene gun etc. and then can be used as a vaccine.

When a vector that grows in the eukaryotic cell is used, it can be introduced per se into eukaryotic cells, for example, lined cell cultures or primary cell cultures, by electroporation, the calcium phosphate method, a method that uses lipofectin, the gene gun method, and the like, and thereby the cells in which said gene has been expressed can be obtained temporarily or permanently by being integrated into the chromosome of the cell. The expressed cells per se or after purification can be used as a component vaccine.

### Examples

The present invention will now be explained with reference to the Examples.

### [Principles of gene alteration]

According to the present invention, when a potential N-glycosylation site of the gene to be expressed in a eukaryotic cell is removed, the following principles A to C were used unless otherwise specified.

### A. Removal of the N-glycosylation site

For the nucleotide sequence corresponding to the potential N-glycosylation site, Asn(N)-X-Ser(S) or Asn(N)-X-Thr(T) (X is an amino acid other than proline), the DNA sequence encoding Asn(N) was modified to a DNA sequence encoding Gln(Q).

### B. Optimization of the vicinity of the ORF initiation codon

Three bases at the 5' end of the ORF initiation codon ATG were modified to AAA, and a total of 6 bases comprising the initiation codon ATG and three bases located upstream thereof were changed to AAAATG. Alteration like this does not badly affect the Kosak rule or the POX rule.

### C. Removal of T5NT

When T5NT that is likely to become the translational termination codon is to be expressed in poxvirus, the base sequence of the region was modified to prevent the termination of translation by taking care not to be accompanied by amino acid substitution.

### D. Optimization to codons frequently used in chickens

Furthermore, optimization to codons frequently used in chickens was also performed as needed. According to the article by Nakamura et al. (1996), basically the most frequently used codon was selected, and the nucleotide sequence was modified so as to make the codon.

### Reference Example 1. Alteration of the MDVgB signal

The DNA sequences corresponding to two N-glycosylation sites present in a 186 bp (62 amino acids) signal sequence of Marek's disease virus gB protein as set forth in SEQ ID NO: 5 were modified based on the above principle of alteration A, and DNA encoding the amino acid sequence as set forth in SEQ ID NO: 6 was obtained.

The DNA was further modified based on the above principle of alteration B and C to obtain the MDVgB signal DNA as set forth in SEQ ID NO: 7 in which the nucleotide sequence was modified.

The modified MDVgB signal DNA was cloned into plasmid PCR-II (Invitrogen) to construct the plasmid PCR2-MDgB-CG. There are a BamHI site on the 5'-end and an EcoRI site on the 3'-end of the modified MDVgB signal DNA in said plasmid.

### Reference Example 2. Alteration of the rabies qG signal

DNA encoding the rabies virus gG signal (23 amino acids) as set forth in SEQ ID NO: 8 having no N-glycosylation sites was modified based on the above principle of alteration B-D to obtain a plasmid pUC-rgG that has an modified rabies gG signal DNA as set forth in SEQ ID NO: 11.

Specific procedure to obtain this plasmid is as follows:

Thus, after annealing two synthetic DNAs as set forth in SEQ ID NO: 9 and 10, it was inserted into a 2665 bp BamHI-EcoRI-cleaved fragment of pUC18 to construct pUC-rgG.

### Example 1. Alteration of the TTM-1 gene from MG

### (1) Construction of pGTPs40KS-Ngly

There are four N-glycosylation sites in the TTM-1 portion of the amino acid sequence as set forth in SEQ ID NO: 12 of a plasmid pNZ40K-S described in International Patent Publication WO97/36924 in which a MDVgB signal-encoding DNA has been ligated to the N-terminal of the antigen gene TTM-1 derived from Mycoplasma gallisepticum. Since there are no N-glycosylation sites in the region from the EcoRI site, the start of the TTM-1 portion, to the BglII site 83 bp downstream, the portion downstream of BglII was modified based on the above principle of alteration A to obtain a plasmid pGTPs40KS-Ngly having an modified TTM-1 gene in which the sequence downstream to the BglII site has the nucleotide sequence as set forth in SEQ ID NO: 24 that corresponds to the amino acid sequence as set forth in SEQ ID NO: 23. The specific procedure to obtain this plasmid is as follows:

By using a synthetic DNA as a primer, mutation was performed using PCR. The PCR used Pfu polymerase (Promega) and was performed under the usual condition using the DNA Thermal Cycler 480 of Perkin-Elmer. Twenty five to thirty cycles were performed at an annealing temperature range of 60°C to 47°C and after determining the optimum condition.

As a template for PCR, pGTPs40K-S having TTM-1 described in WO97/36924 and the MDVgB signal was used.

First, PCR was performed with the primer 40KG-1 (SEQ ID NO: 13) and the primer 40KG-2R (SEQ ID NO: 14) to obtain a 136 bp fragment.

Similarly, PCR was performed with the primer 40KG-2 (SEQ ID NO: 15) and the primer 40KG-3R (SEQ ID NO: 16) to obtain a 341 bp fragment.

Similarly, PCR was performed with the primer 40KG-3A (SEQ ID NO: 17) and the primer 40KG-4RA (SEQ ID NO: 18) to obtain a 190 bp fragment.

Similarly, PCR was performed with the primer 40KG-4 (SEQ ID NO: 19) and the primer 40KG-5R (SEQ ID NO: 20) to obtain a 359 bp fragment.

Similarly, PCR was performed with the primer 40KG-5(SEQ ID NO: 21) and the primer 40KG-6R (SEQ ID NO: 22) to obtain a 218 bp fragment.

In the next PCR, using three fragments of a 136 bp PCR product of the primers 40KG-1 and 40KG-2R, a 341 bp PCR product of the primers 40KG-2 and 40KG-3R, and a 190 bp PCR product of the primers 40KG-3A and 40KG-4RA as the templates, PCR was performed in the above-mentioned condition with the primer 40KG-1 (SEQ ID NO: 13) and the primer 4KG-4RA (SEQ ID NO: 18) to obtain a 595 bp fragment.

Similarly, using a 359 bp PCR product of the primers 40KG-4 and 40KG-5R and a 218 bp PCR product of the primers 40KG-5 and 40KG-6R as the templates, PCR was performed in the above-mentioned condition with the primer 40KG-4 (SEQ ID NO: 19) and the primer 4KG-6R (SEQ ID NO: 22) to obtain a 539 bp fragment.

Furthermore, using a 595 bp PCR product of the primers 40KG-1 and 40KG-4RA and a 539 bp PCR product of the primers 40KG-4 and 40KG-6R as the templates, PCR was performed in the above-mentioned condition with the primer 40KG-1 (SEQ ID NO: 13) and the primer 4KG-6R (SEQ ID NO: 22) to obtain a partial fragment (1088 bp) of the modified TTM-1.

By ligating a fragment obtained by cleaving this 1088 bp fragment with BglII and SalI and a 2896 bp obtained by cleaving pGTPs40K-S described in WO97/36924 with BglII and SalI, the plasmid pGTPs40KS-Ngly having an modified TTM-1 gene in which the DNA region encoding the N-glycosylation site has been modified was constructed.

### Example 2. Alteration of the mgc3 gene derived from MG

The amino acid sequence as set forth in SEQ ID NO: 25 encoded by the mgc3 gene (GeneBank accession No. AB023292) as set forth in SEQ ID NO: 2 has 16 N-glycosylation sites. Among the 16 sites, since a site at the most 5' upstream side is replaced with the signal sequence at a later treatment, it was excluded from the target of alteration. For the remaining 15 sites, the base sequence was modified based on the principle of alteration A to obtain the plasmid pM11BTR containing the mgc3 gene that has the nucleotide sequence as set forth in SEQ ID NO: 79 corresponding to the amino acid sequence as set forth in SEQ ID NO: 78.

The specific procedure to obtain this plasmid is as follows:

For the alteration, a synthetic DNA was used as the primer, and mutation was effected by PCR.

Since the mgc3 gene is as long as about 3 kb, it was divided into three fragments of about 1 kb in length, which were termed as the 1094 bp BKR region, the 908 bp KXR region, and the 1192 bp XGTR region. For each of these three fragments, mutation was effected, and after confirming the nucleotide sequence, they were ligated.

Using Pfu polymerase (Promega) as in Example 1, PCR was performed under the usual condition. Twenty five to thirty cycles were performed at an annealing temperature range of 60°C to 47°C and after determining the optimum condition. As the template for the initial alteration, pUC-MGC3 in which the mgc3 gene derived from Mycoplasma gallisepticum described in Yoshida et al. (2000) was inserted into pUC18 was used.

### (1) Mutation of the BKR region (construction of pM11BTR)

Using pUC-MGC3 as the template, PCR was performed with the primer M11-B (SEQ ID NO: 26) and the primer M11-2R (SEQ ID NO: 27) to obtain a 136 bp fragment.

Using pUC-MGC3 as the template, PCR was performed with the primer M11-2 (SEQ ID NO: 28) and the primer M11-3R (SEQ ID NO: 29) to obtain a 92 bp fragment.

Using pUC-MGC3 as the template, PCR was performed with the primer M11-3 (SEQ ID NO: 30) and the primer M11-4RB (SEQ ID NO: 31) to obtain a 271 bp fragment.

Using pUC-MGC3 as the template, PCR was performed with the primer M11-4B (SEQ ID NO: 32) and the primer M11-5R (SEQ ID NO: 33) to obtain a 116 bp fragment.

Using pUC-MGC3 as the template, PCR was performed with the primer M11-5A (SEQ ID NO: 34) and the primer M11-7RA (SEQ ID NO: 35) to obtain a 439 bp fragment.

Using pUC-MGC3 as the template, PCR was performed with the primer M11-7 (SEQ ID NO: 36) and the primer M11-KR (SEQ ID NO: 37) to obtain a 201 bp fragment.

In the next PCR, using a 136 bp PCR product of the primer M11-B and the primer M11-2R and a 92 bp PCR product of the primer M11-2 and the primer M11-3R as the templates, PCR was performed with the primer M11-B (SEQ ID NO: 26) and the primer M11-3R (SEQ ID NO: 29) to obtain a 199 bp fragment.

Similarly, using a 271 bp PCR product of the primer M11-3 and the primer M11-4RB and a 116 bp PCR product of the primer M11-4B and the primer M11-5R as the templates, PCR was performed with the primer M11-3 (SEQ ID NO: 30) and the primer M11-5R (SEQ ID NO: 33) to obtain a 344 bp fragment.

Similarly, using a 439 bp PCR product of the primer M11-5A and the primer M11-7RA and a 201 bp PCR product of the primer M11-7 and the primer M11-KR as the templates, PCR was performed with the primer M11-5A (SEQ ID NO: 34) and the primer M11-KR (SEQ ID NO: 37) to obtain a 610 bp fragment.

Using these three PCR products, a PCR product (199 bp) of the primer M11-B and the primer M11-3R, and a PCR product (361 bp) of the primer M11-3 and the primer M11-5R, and a PCR product (610 bp) of the primer M11-5A and M11-KR as the templates, PCR was performed with the primer M11-B (SEQ ID NO: 26) and the primer M11-KR (SEQ ID NO: 37) to obtain a 1094 bp fragment. A 1070 bp fragment obtained by cleaving this PCR product (1094 bp) of the primers M11-B and M11-KR with EcoRI and KpnI was inserted into a 2678 bp fragment of the plasmid pUC18 cleaved with EcoRI and KpnI to construct pM11BKR.

After analyzing the nucleotide sequence of this pM11BKR, a sequence different from the mgc3 gene (GeneBank accession No. AB023292) registered in GeneBank was found. Furthermore, the sequence of the original plasmid pUC-MGC3 was compared to confirm that it was not an error in PCR. As a result, it was confirmed that G at position 308 of SEQ ID NO: 2, a sequence registered at GeneBank, is T, G at position 311 is C, C at position 561 is G, and G at position 749 is T. It was demonstrated that due to an error in the base at position 561, the amino acid sequence encoded in this region is not N-Asn(N)-Gln(Q)-Thr(T) corresponding to the N-glycosylation site but Gln(Q)-Gln(Q)-Thr(T).

### (2) Mutation of the KXR region (construction of pM11KXR)

Using pUC-MGC3 as the template, PCR was performed with the primer M11-K (SEQ ID NO: 38) and the primer M11-8R (SEQ ID NO: 39) to obtain a 151 bp fragment.

Using pUC-MGC3 as the template, PCR was performed with the primer M11-8 (SEQ ID NO: 40) and the primer M11-10R (SEQ ID NO: 41) to obtain a 109 bp fragment.

Using pUC-MGC3 as the template, PCR was performed with the primer M11-10 (SEQ ID NO: 42) and the primer M11-12RA (SEQ ID NO: 43) to obtain a 416 bp fragment.

Using pUC-MGC3 as the template, PCR was performed with the primer M11-12A (SEQ ID NO: 44) and the primer M11-XR (SEQ ID NO: 45) to obtain a 350 bp fragment.

In the next PCR, using a 109 bp PCR product of the primer M11-8 and the primer M11-10R and a 416 bp PCR product of the primer M11-10 and the primer M11-12RA as the templates, PCR was performed with the primer M11-8 (SEQ ID NO: 40) and the primer M11-12RA (SEQ ID NO: 43) to obtain a 487 bp fragment.

Furthermore, using a 151 bp PCR product of the primer M11-K and the primer M11-8R and a 487 bp PCR product of the primer M11-8 and the primer M11-12RA as the templates, PCR was performed with the primer M11-K (SEQ ID NO: 38) and the primer M11-12RA (SEQ ID NO: 43) to obtain a 596 bp fragment.

Using the above two PCR products, a 596 bp PCR product of the primer M11-K and the primer M11-12RA and a 350 bp PCR product of the primer M11-12A and the primer M11-XR as the templates, PCR was performed with the primer M11-K (SEQ ID NO: 38) and the primer M11-XR (SEQ ID NO: 45) to obtain a 908 bp fragment.

A 885 bp fragment obtained by cleaving this PCR product (908 bp) of the primer M11-K and the primer M11-XR with KpnI and XbaI was inserted into the plasmid pUC18 cleaved with KpnI and XbaI to construct pM11KXR.

After analyzing the nucleotide sequence of this pM11KXR, a sequence different from the mgc3 gene (GeneBank accession No. AB023292) registered in GeneBank was found. Furthermore, the sequence of the original plasmid pUC-MGC3 was compared to confirm that it was not an error in PCR. As a result, it was confirmed that G at position 1279 of SEQ ID NO: 2, a sequence registered at GeneBank, is A, T at position 1729 is G, and C at position 1732 is G.

### (3) Mutation of the XGTR region (construction of pM11XGTR)

Using pUC-MGC3 as the template, PCR was performed with the primer M11-XA (SEQ ID NO: 46) and the primer M11-13RA (SEQ ID NO: 47) to obtain a 238 bp fragment.

Using pUC-MGC3 as the template, PCR was performed with the primer M11-13A (SEQ ID NO: 48) and the primer M11-14RA (SEQ ID NO: 49) to obtain a 266 bp fragment.

Using pUC-MGC3 as the template, PCR was performed with the primer M11-14A (SEQ ID NO: 50) and the primer M11-15RA (SEQ ID NO: 51) to obtain a 168 bp fragment.

Using pUC-MGC3 as the template, PCR was performed with the primer M11-15A (SEQ ID NO: 52) and the primer M11-16RA (SEQ ID NO: 53) to obtain a 123 bp fragment.

Using pUC-MGC3 as the template, PCR was performed with the primer M11-16A (SEQ ID NO: 54) and the primer M11-GTR (SEQ ID NO: 55) to obtain a 556 bp fragment.

In the next PCR, using a 238 bp PCR product of the primer M11-XA and the primer M11-13RA and a 266 bp PCR product of the primer M11-13A and the primer M11-14RA as the templates, PCR was performed with the primer M11-XA (SEQ ID NO: 46) and the primer M11-14RA (SEQ ID NO: 49) to obtain a 463 bp fragment.

Furthermore, using a 168 bp PCR product of the primer M11-14A and the primer M11-15RA and a 123 bp PCR product of the primer M11-15A and the primer M11-16RA as the templates, PCR was performed with the primer M11-14A (SEQ ID NO: 50) and the primer M11-16RA (SEQ ID NO: 53) to obtain a 253 bp fragment.

Using the above three fragments of a 463 bp PCR product of the primer M11-XA and the primer M11-14RA, a 253 bp PCR product of the primer M11-14A and the primer M11-16RA, and a 556 bp PCR product of the primer M11-16A and the primer M11-GTR as the templates, PCR was performed with the primer M11-XA (SEQ ID NO: 46) and the primer M11-GTR (SEQ ID NO: 55) to obtain a 1192 bp fragment.

A 1174 bp fragment obtained by cleaving this PCR product (1192 bp) of the primer M11XA and the primer M11-GTR with XbaI and SalI was inserted into a 2680 bp fragment of the plasmid pUC18 cleaved with XbaI and SalI to construct pM11XGTR.

After analyzing the nucleotide sequence of this pM11XGTR, a sequence different from the mgc3 gene (GeneBank accession No. AB023292) registered in GeneBank was found. Furthermore, the sequence of the original plasmid pUC-MGC3 was compared to confirm that it was not an error in PCR. As a result, it was confirmed that G at position 3113 of SEQ ID NO: 2, a sequence registered at GeneBank, is C.

### (4) Construction of the plasmid pM11BTR containing the sugar chain-removed full-length mgc3

A 885 bp fragment prepared by cleaving pM11KXR with KpnI and XbaI and a 1174 bp fragment prepared by cleaving pM11GTR with XbaI and SalI were ligated to a 3723 bp fragment prepared by cleaving pM11BKR with KpnI and SalI thereby to construct pM11BTR having the modified mgc3 gene in which the DNA region encoding a N-glycosylation site was modified.

### Example 3. Preparation of vectors for recombinant fowlpox virus

### (1) Construction of pUCSfi-H-S

A 2676 bp fragment obtained by cleaving, with HindIII and SalI, pUC18XG described in International Patent Publication WO99/18215 in which the restriction sites of pUC18 were modified and an annealing product of a 5'-end-phosphorylated linker H'-S-H-S-P-S1 (SEQ ID NO: 56) and a linker H'-S-H-S-P-S2 (SEQ ID NO: 57) were ligated to construct pUCSfi-H-S.

### (2) Construction of pGHPs

Three fragments, a 2661 bp fragment obtained by cleaving pUCSfi-H-S with HindIII and EcoRI, an annealing product of a 5'-end-phosphorylated linker S-B-E1 (SEQ ID NO: 58) and a linker S-B-E2 (SEQ ID NO: 59), and a 137 bp fragment obtained by cleaving, with HindIII and SalI, the plasmid described in International Patent Publication WO97/36924 containing the poxvirus late and early promoter, were ligated to construct a plasmid pGHPs.

### (3) Construction of pGTPs40KS(CG1)

A plasmid PCR2-MDgB-CG having the MDVgB signal DNA modified in Reference Example 1 was cleaved with BamHI and EcoRI to obtain a 189 bp fragment.

pGTPs40K-S having the MDVgB signal DNA described in International Patent Publication WO97/36924 and the TTM-1 gene were cleaved with BamHI and EcoT22I to collect a 3402 bp DNA fragment. Similarly, pGTPs-40K-S was cleaved with EcoRI and EcotT22I to collect a 557 bp DNA fragment. The three fragments thus obtained were ligated to construct a plasmid pGTPs40KS(CG1).

### (4) Construction of pGTPs40K-G-CS

A 3067 bp fragment obtained by cleaving the plasmid pGTPs40K-Ngly having the modified TTM-1 gene obtained in Example 1 with BglII and SalI and a 1082 bp fragment obtained by cleaving pGTPs40KS(CG1) obtained in the above (3) with BglII and SalI were ligated to construct a plasmid pGTPs40K-G-CS.

### (5) Construction of pNZ1829R/40K-G-CS

A 9217 bp fragment obtained by cleaving a plasmid pNZ1829R described in WO97/36924 containing a sequence for homologous recombination of FPV, the late promoter and the early promoter of pox, and a marker gene, lacZ gene, with BamHI and SalI, and a 1347 bp fragment obtained by cleaving pGTPs40K-G-CS obtained in the above (4) with BamHI and SalI were ligated to construct a plasmid pNZ1829R/40K-G-CS having an modified TTM-1 gene in which the modified MDVgB signal DNA was connected in frame and the lacZ gene as a marker gene.

### (6) Construction of pNZ1829R/40K-G-CS(dl-lacZ)

A 7013 bp fragment obtained by cleaving this plasmid pNZ1829R/40K-G-CS with SmaI and SfiI was treated with T4 polymerase, blunted, and the fragment obtained was subjected to self-ligation to construct a plasmid pNZ1829R/40K-G-CS (dl-lacZ) in which the lacZ gene was deleted from the plasmid pNZ1829R/40K-G-CS. By sequencing, the nucleotide sequence of the blunted portion was confirmed.

### Example 4. Construction of vectors for recombinant fowlpox virus

### (1) Construction of pGHPs40KCS-G

A 1347 bp fragment obtained by cleaving pGTPs40K-G-CS constructed in Example 3(4) with BamHI and SalI and a 2791 bp fragment obtained by cleaving pGHPs constructed in Example 3(2) with BamHI and SalI were ligated to construct pGHPs40KCS-G.

### (2) Construction of pNZ29RMG40KM11CS-G

A 3129 bp fragment obtained by cleaving pM11BTR having the modified mgc3 gene constructed in Example 2(4) with EcoRI and SalI, a 297 bp fragment obtained by cleaving pGHPs40KCS-G obtained in the above (1) with EcoRI and SfiI, and a 6985 bp fragment obtained by cleaving pNZ1829R/40K-G-CS constructed in Example 3(5) with SfiI and SalI were ligated to construct a plasmid pNZ29RMG40KM11CS-G having the modified MDVgB signal DNA, the modified TTM-1 gene, and the modified mgc3 gene.

### Example 5. Construction of a vector pNZ29RMG40KM11CS-G2 for recombinant fowlpox virus

In Example 4, the modified mgc3 gene has been inserted in the constructed pNZ29RMG40KM11CS-G. In order to return the site that, inherently, is not a N-glycosylation site (corresponding to base at position 561 in SEQ ID NO: 2) of the gene to the original base G, this part was further modified, and a plasmid pNZ29RMG40KM11CS-G2 having the re-modified mgc3 gene was obtained.

The specific procedure used for re-altering mgc3 is as follows:

Using pNZ29RMG40KM11CS-G constructed in Example 4(2) as the template, PCR was performed with the primer M11-Sfi (SEQ ID NO: 60) and the primer M11-5RB (SEQ ID NO: 61) in the condition described in Example 1 to obtain a 836 bp fragment.

Using pNZ29RMG40KM11CS-G constructed in Example 4(2) as the template, PCR was performed with the primer M11-5C (SEQ ID NO: 62) and the primer M11-KRA (SEQ ID NO: 63) in a similar condition to obtain a 618 bp fragment.

Using these two PCR products as the templates, PCR was performed with the primer M11-Sfi (SEQ ID NO: 60) and the primer M11-KRA (SEQ ID NO: 63) to obtain a 1400 bp fragment. A 1368 bp fragment obtained by cleaving this 1400 bp PCR product with SfiI and KpnI and a 9032 bp fragment obtained by cleaving pNZ29RMG40KM11CS-G constructed in Example 4(2) with SfiI and KpnI were ligated to construct a plasmid pNZ29RMG40KM11CS-G2 having the modified MDVgB signal DNA, the modified TTM-1 gene, and the re-modified mgc3 gene.

By sequencing the modified part, the nucleotide sequence was confirmed.

### Example 6. Construction of vectors for recombinant HVT

### (1) Construction of pGIPec40KS

A 3278 bp fragment obtained by cleaving the plasmid pGIPec described in Japanese Unexamined Patent Publication (Kokai) No. 2001-188 having the Pec promoter and the polyA signal (Yanagida et al., 1993) of UL46h, UL47h and UL49h with BamHI and SalI and a 1344 bp fragment obtained by cleaving pGTPs40K-G-CS constructed in Example 3(4) with BamHI and SalI fragments were ligated to construct a plasmid pGIPec40KS.

### (2) Construction of p45/46MG40KS

To the SfiI site of the plasmid pNZ45/46Sfi described in International Patent Publication WO99/18215 having the homologous recombination site of UL44-UL46 of HVT, a BglI fragment (1969 bp) of pGIPec40KS obtained in the above (1) was inserted to construct a plasmid p45/46MG40KS.

### (3) Construction of pGICMV(-)

Using pGIPec described in Japanese Unexamined Patent Publication (Kokai) No. 2001-188 as the template, PCR was performed in the condition created in Example 1 with the primer pCMV-1 (SEQ ID NO: 64) and the primer pPec1R (SEQ ID NO: 65) to obtain a 293 bp fragment.

Using pBK-CMV (Stratagene) as the template, PCR was performed in a similar condition with the primer pCMV-o1 (SEQ ID NO: 66) and the primer CMV-R1 (SEQ ID NO: 67) to obtain a 341 bp fragment.

Using the two PCR products obtained as the templates, PCR was performed with the primer pCMV-1 (SEQ ID NO: 64) and the primer pCMV-R1 (SEQ ID NO: 67) to obtain a 604 bp fragment.

A 589 bp fragment obtained by cleaving this 604 fragment with PstI and XbaI and a 2765 bp fragment obtained by cleaving pGIPec with PstI and XbaI were ligated to construct pGICMV(-).

### (4) Construction of pGHMCSpolyASfi

Using the plasmid pGIMCSpolyASfi described in International Patent Publication WO99/18215 having the polyA signal of UL46h, UL47h and UL49h and a multiple cloning site as the template, PCR was performed with the primer pGHP-F (SEQ ID NO: 68) and the primer pGHP-R (SEQ ID NO: 69) to obtain a 149 bp fragment.

A 138 bp fragment obtained by cleaving this 149 bp fragment with EcoRI and HindIII and a 2635 bp fragment obtained by cleaving pGIMCSpolyASfi with EcoRI and HindIII were ligated to obtain pGHMCSpolyASfi.

### (5) Construction of pGHCMV(-)

A 2765 bp fragment obtained by cleaving pGHMCSpolyASfi obtained in the above (4) with PstI and XbaI and a 589 bp fragment obtained by cleaving pGICMV(-) obtained in the above (3) with PstI and XbaI were ligated to construct pGHCMV(-).

### (6) Construction of pHCMV(-)

A 3294 bp fragment obtained by cleaving pGHCMV(-) obtained in the above (5) with EcoRI and SalI and an annealing product of 5'-end-phosphorylated Linker 1 (SEQ ID NO: 70) and a Linker 2 (SEQ ID NO: 71) were ligated to construct a plasmid pHCMV(-) in which the polyA signal of UL46h, UL47h, and UL49h of MDV-1 was deleted in pGHCMV(-).

### (7) Construction of pHCMV-M11 (CSG2)

A 3275 bp fragment obtained by cleaving pHCMV(-) obtained in the above (6) with BamHI and SalI and a 3318 bp fragment obtained by cleaving pNZ29RMG40KM11CS-G2 obtained in Example 5 with BamHI and SalI were ligated to construct a plasmid pHCMV-M11 (CSG2).

### (8) Construction of pBAc

A 1515 bp fragment obtained by cleaving a plasmid pLUC-bac having the chicken beta-actin promoter described in Japanese Unexamined Patent Publication (Kokai) No. 2001-188 with PstI and XbaI and a 2765 bp fragment obtained by cleaving pGIMCSpolyASfi described in International Patent Publication WO99/18215 with PstI and XbaI were ligated to construct a plasmid pBAc.

### (9) Construction of pBAc (dHS)

A 4268 bp fragment obtained by cleaving a plasmid pBAc obtained in the above (8) with XbaI after partially cleaving with HindIII and an annealing product of 5'-end-phosphorylated Linker 3 (SEQ ID NO: 72) and a Linker 4 (SEQ ID NO: 73) were ligated to construct a plasmid pBAc(dHS).

### (10) Construction of pGIBacpA2nd

Using pBK-CMV (Stratagene) as the template, PCR was performed with the primer PolyA-SfiF2 (SEQ ID NO: 74) and the primer PolyA-SalKpn (SEQ ID NO: 75) to obtain a 313 bp fragment having the PolyA signal of SV40. A 297 bp fragment obtained by cleaving this 313 bp fragment with SfiI and Kpn and a 4222 bp fragment obtained by cleaving pBAc(dHS) obtained in the above (9) with SfiI and KpnI were ligated to construct pGIBacpA2nd.

### (11) Construction of pGIBac40KS2nd

Using pGTPs40K-S described in International Patent Publication WO97/36924 as the template, PCR was performed with the primer 40KS-B (SEQ ID NO: 76) and 40KG-6R (SEQ ID NO: 22) to obtain a 1359 bp fragment. A 1345 bp fragment obtained by cleaving this 1359 bp fragment with BamHI and SalI and a 4222 bp fragment obtained by cleaving pGIBacpA2nd obtained in the above (10) with BamHI and SalI were ligated to construct a plasmid pGIBac40KS2nd.

### (12) Construction of p45/46Bac40KS+2nd

A 3169 bp fragment obtained by cleaving pGIBac40KS2nd obtained in the above (11) with BglI was inserted into the SfiI site of pNZ45/46Sfi having the UL44 to UL46 region (SEQ ID NO: 3) of HVT described in WO97/36924 to construct a plasmid p45/46Bac40KS+2nd having the TTM-1 gene.

### (13) Construction of p45/46Bac40KS-CMVM11

A 3940 bp fragment obtained by cleaving pHCMV-M11(CSG2) obtained in the above (7) with BglI was inserted into the SfiI site of p45/46Bac40KS+2nd obtained in the above (12) to construct a plasmid p45/46Bac40KS-CMVM11 having the mgc3 gene.

### (14) Construction of p45/46Pec40KS+2nd

A 561 bp fragment obtained by cleaving pGIPec40KS obtained in the above (1) with PstI and BamHI, a 3520 bp fragment obtained by cleaving p45/46Bac40KS+2nd obtained in the above (12) with BamHI and HindIII, and a 3613 bp fragment obtained by cleaving the same plasmid with PstI and HindIII were ligated to construct a plasmid p45/46Pec40KS+2nd having the modified MDVgB signal DNA and the modified TTM-1 gene.

### (15) Construction of pNZ45/46BacpA2nd

A 1866 bp fragment obtained by cleaving pGIBacA2nd obtained in the above (11) with BglI was inserted into the SfiI site of pNZ45/46Sfi described in WO99/18215 to construct pNZ45/46BacpA2nd.

### (16) Construction of pNZ45/46Bac40KpA+2nd

Using pUTTM-1 described in U.S. Pat. No. 5,489,430 as the template, PCR was performed with primer pMG40K-1 (SEQ ID NO: 77) and the primer 40KG-6R (SEQ ID NO: 22) to obtain a 1259 fragment. A 1237 bp fragment obtained by cleaving this 1259 bp fragment with BamHI and SalI and a 7317 bp fragment obtained by cleaving pNZ45/46BacpA2nd obtained in the above (15) with BamHI and SalI were ligated to construct a plasmid pNZ45/46Bac40KpA+2nd.

### (17) p45/46Bac40K-CMVM11

A 3940 bp fragment obtained by cleaving pHCMV-M11(CSG2) obtained in the above (7) with BglI was inserted into the SfiI site of pNZ45/46Bac40KpA2nd obtained in the above (16) to construct a plasmid p45/46Bac40K-CMVM11 having the modified MDVgB signal DNA, the modified TTM-1 gene, and the modified mgc3 gene.

### Example 7. Construction of vectors for recombinant fowlpox virus

### (1) Construction of pNZ29R/40KMGC3

A 1345 bp fragment obtained by cleaving pGIBac40KS2nd constructed in Example 6(11) with BamHI and SalI, a 1333 bp fragment obtained by cleaving pNZ29RMG40KM11CS-G2 constructed in Example 5 with EcoT22I and BamHI, and a 7722 bp fragment obtained by cleaving the same plasmid with EcoT22I and SalI were ligated to construct a plasmid pNZ29R/40KMGC3 that is a vector for recombinant fowlpox virus having the modified MDVgB signal DNA, the modified TTM-1 gene, and the re-modified mgc3 gene.

### (2) Construction of pNZ29RMG40KSM11CS-G2

A 1440 bp fragment obtained by cleaving pGTPs40KS-Ngly constructed in Example 1 with HindIII and SalI, a 1237 bp fragment obtained by cleaving pNZ29RMG40KM11CS-G2 constructed in Example 5 with HindIII and EcoT22I, and a 7722 bp fragment obtained by cleaving the same plasmid with EcoT22I and SalI were ligated to construct a plasmid pNZ29RMG40KSM11CS-G2 that is a vector for recombinant fowlpox virus having the modified MDVgB signal DNA, the modified TTM-1 gene, and the re-modified mgc3 gene.

### (3) Construction of pGHPs-rgG

A 75 bp fragment obtained by cleaving pUC-rgG constructed in Reference Example 2 with BamHI and EcoRI and a 2756 bp fragment obtained by cleaving pGHPs40KCS-G constructed in Example 4 with BamHI and EcoRI were ligated to construct a plasmid pGHPs-rgG.

### (4) Construction of pNZ29RMG40KSM11(rgG)CS-G2

A 3129 bp fragment obtained by further cleaving, with EcoRI, a 3418 bp fragment obtained by cleaving pNZ29R/40KMGC3 obtained in the above (1) with SfiI and SalI, a 6982 bp fragment obtained by cleaving the same pNZ29R/40KMGC3 with SfiI and SalI, and a 176 bp fragment obtained by cleaving pGHPs-rgG constructed in the above (3) with SfiI and EcoRI were ligated to construct a plasmid pNZ29RMG40KSM11(rgG)CS-G2 having that is a vector for recombinant fowlpox virus having the modified rgG gene, the modified TTM-1 gene, and the re-modified mgc3 gene.

### Example 8. Purification of recombinant fowlpox virus

To a monolayer of CEFs, the BLEN strain (FP-Blen Select Laboratories) that is a live vaccine for fowl pox was infected at a M.O.I. of 0.1. Three hours later, these cells were trypsinized to make a cell suspension. The cells (2 x 10⁷ cells) in this suspension and 10 µg of one of the vectors for recombinant fowlpox virus shown in Table 1 constructed in the above Examples were mixed, and the mixture was suspended into Saline G (0.14 M NaCl, 0.2 mM KCl, 1.1 mM hydrogen disodium phosphate, 1.5 mM hydrogen potassium phosphate, 0.5 mM magnesium chloride hexahydrate, 0.011% glucose), which was then subjected to electroporation at a condition of 3.0 KVcm⁻¹ and 0.4 msec at room temperature using the Gene Pulser (Bio-Rad). Cells into which the vector for recombinant fowlpox virus had been introduced were then cultured for 72-120 hours, freeze-thawed 3 times, and the cells containing recombinant fowlpox virus were collected. The relationship between the vector for recombinant fowlpox virus and the name of the recombinant fowlpox virus obtained from the vector is shown in Table 1.

**Table 1: The relationship between the recombinant fowlpox virus and the recombinant vector**

| Vector for recombinant fowlpox virus | recombinant fowlpox virus |
|---|---|
| pNZ1829R/40K-S (dl-lacZ) | rFPV-B/MG40- |
| pNZ29RMG40KM11CS-G | rFPV-B/MG40/M11 |
| pNZ1829R/40K-G-CS | rFPV-B/MG40-S |
| pNZ29R/40KMGC3 | rFPV-B/MG-1 |
| pNZ29RMG40KS(-G)M11CS-G2 | rFPV-B/MG-2 |
| pNZ29RMG40KSM11(rgG)CS-G2 | rFPV-B/MG-3 |

The recovered virus was purified by the Black Plaque Assay (BPA).

The solution of the recovered virus was infected to the monolayer of CEFs, on which agar containing the growth medium was overlaid. After confirming virus plaques 2-3 days later, each virus plaque was collected individually with the agar on it with a Pasteur pipet etc., suspended in the growth medium, and stored. On the other hand, agar was removed from CEFs having the remaining plaques, which was then fixed in cold methanol etc. Antiserum (anti-TTMG-1 antiserum) obtained by immunizing rabbits with the TTMG-1 protein that was expressed in Escherichia coli was diluted about 500-fold in Dulbecco's phosphate buffered saline (Dainippon Pharmaceutical; hereinafter referred to as PBS(-)) which is usually used for cell culture and includes no magnesium ion, and was reacted to the plaques at 22-25°C for 2 hours. The antibody that was not bound was washed three times in 3% non-fat dried milk in PBS(-), and then, biontinylated anti-rabbit antibody (goat, Biosource) was reacted to the plaque at 22-25°C for 2 hours.

After the reaction was over the antibody was washed out by PBS(-), avidin-biotin-alkaline phosphatase complex (Vector Laboratories) was reacted. After rinsing off the unreacted avidin-biotin-alkaline phosphatase complex in PBS(-), plaques were developed with the substrate for alkaline phosphatase, BCIP/NBT (Roche), a which creates dark blue or black precipitate. Using this BPA, viral suspensions corresponding to positive plaques were re-infected to CEFs and similar procedures were repeated for 3-4 times until all plaques became positive with BPA. The gene structure of recombinants was confirmed by Southern hybridization and the sequencing of the DNA sequence at the junction.

### Example 9. Purification of recombinant HVT

According to the method of Morgan et al. (1990), HVT-DNA was recovered. The specific method of collection is as follows:

About 10⁵ PFU of the HVT FC126 strain (ATCC VR-584B) was infected to about 3 x 10⁷ CEFs. After culturing for 2-3 days, 4 ml of the lysis buffer (0.5% SDS, 10 mM Tris (pH 8.0), 100 mM NaCl, 1 mM EDTA, 200 µg/ml Proteinase K) was added thereto, and incubated at 37°C for 4 hours, phenol-extracted, and precipitated with ethanol to recover HVT-DNA.

The vectors for recombinant HVTs shown in Table 2 obtained in the above Examples were cleaved with an appropriate restriction enzyme into linearized at the site which is located on neither the homologous region to HVT genome nor the foreign gene(s).

About 3 x 10⁶ CEFs collected by trypsinization were suspended in saline G (0.14 M NaCl, 0.2 mM KCl, 1.1 mM hydrogen disodium phosphate, 1.5 mM hydrogen potassium phosphate, 0.5 mM magnesium chloride hexahydrate, 0.011% glucose). 10-30 µg of the previously prepared HVT-DNA and 10-30 µg of the linearized vector for recombination were introduced into the cells by electroporation at a condition of 0.5 KVcm⁻¹ and 0.4 msec at room temperature using Gene Pulser (Bio-Rad). This cell suspension was plated on a 6-cm culture dish, to which the growth medium was added and cultured for 5-7 days. The infected cells which contained recombinant HVTs were harvested. The recombinant HVT was purified by the limiting dilution method. The specific method of purification is as follows:

About 2 x 10⁶ CEFs together with the serially diluted viral solutions were plated on 96-well plates. After culturing for 3-5 days when plaques appeared, replica was made. From one of the plates, using a similar method to the BPA method in Example 8, recombinant HVTs that expressed the TTM-1 gene were selected. In a similar manner to recombinant fowlpox virus, the viral solutions of the replica corresponding to positive plaques were re-infected to CEFs, and similar procedures were repeated for 3-4 times until all plaques became positive by the BPA method.

The relationship between the vectors for HVT and the recombinant HVTs obtained from the vector is shown in Table 2.

**Table 2: The relationship between the name of the recombinant HVT and the recombinant vector**

| Vector for recombinant HVT | recombinant HVT |
|---|---|
| p45/46MG40KS | rHVT/PecMG40KS |
| p45/46Bac40KS-CMVM11 | rHVT/Bac40KS-CMVM11 |
| p45/46Pec40KpA+2nd | rHVT/PecMG40KS+2nd |
| p45/46Bac40K-CMVM11 | rHVT/Bac40K-CMVM11 |

The gene structure of recombinants was confirmed by Southern hybridization and the sequencing of the DNA sequence at the junction.

### Example 10. Confirmation of the protein expressed from the recombinants (the BPA method)

It has already been confirmed by the BPA method in each Example that all of the recombinant fowlpox viruses and the recombinant HVTs obtained in Examples 8 and 9 have the TTM-1 gene or the modified TTM-1 gene and express the TTMG-1 antigen.

For recombinant viruses having the mgc3 gene or the modified mgc3 gene obtained in Examples 8 and 9, rFPV-B/MG40/M11, rFPV-B/MG-1, rFPV-B/MG-2, rFPV-B/MG-3, rHVT/Bac40KS-CMVM11, and rHVT/Bac40K-CMVM11, the BPA method was performed as in Example 8 using mouse monoclonal antibody Mab 35A6 raised against the MGC3 antigen prepared by Yoshida et al. (2000) (however, it differs in that the second antibody is a biotinylated anti-mouse antibody (goat, Biosource)) to confirm the expression of the MGC3 antigen.

### Example 11. Confirmation of the protein expressed from the recombinants (the Western blotting method)

The molecular weight of recombinant virus was determined using the Western blotting method. The procedure by the Western blotting method is as follows:

The monolayer of CEFs infected with recombinant virus was solubilized in the SDS-GEL loading buffer. For some samples, sugar chains were digested as they are using sugar chain-digesting enzymes, endoglycosidase H (Endo H; Roche) and PNGase F (Endo F; Roche). These samples were subjected to SDS-PAGE electrophoresis under the normal denaturing reduced condition. Protein was transferred from the gel to the PVDF membrane (Immobilon-P, Millipore). The PVDF membrane was incubated with the anti-TTMG-1 antiserum or Mab 35A6 described in Example 10, and rinsed in Tris buffered saline with Tween 20 (T-TBS: 0.1 M Tris-Cl (pH 7.5), 0.9% NaCl, 0.1% Tween 20 (manufactured by Sigma-Aldrich)). The membrane was incubated with a second antibody (anti-rabbit (goat) for anti-TTM-1 antiserum, and anti-mouse (goat) for Mab 35A6). After rinsing with T-TBS, it was reacted with the avidin-biotin-alkaline phosphatase complex (Vector Laboratories). By rinsing off the unreacted avidin-biotin-alkaline phosphatase complex in T-TBS, protein bands were observed after development with the substrate for alkaline phosphatase, BCIP/NBT (Roche), which creates dark blue or black precipitate.

It was confirmed by the Western blotting method that the recombinant fowlpox virus rFPV-B/MG40/M11 (Lane 3) obtained in Example 8 expresses the TTMG-1 antigen (Figure 1). As the control, the parent fowlpox virus BLEN strain (Lane 1), and a recombinant fowlpox virus in which the MDVgB signal was connected in frame to the TTMG-1 antigen having a sugar chain described in International Patent Publication WO97/36924, 40K-S (Lane 2), and the recombinant fowlpox virus recFPV-MGC3 (Lane 4) that expresses the MGC3 antigen reported by Yoshida et al. (2000) were used.

As shown in Figure 1, 40K-S (Lane 2) that expresses the TTMG-1 antigen having a N-glycosylation site expressed an about 60 kd TTMG-1 antigen. On the other hand, rFPV-B/MG40/M11 (Lane 3) that expresses the TTMG-1 antigen in which the N-glycosylation site was modified expressed an about 50 kd TTMG-1 antigen. This is consistent with the value of 48.9 kd predicted from the amino acid sequence in which the MDVgB signal was added to the N-glycosylation site-modified TTM-1. In the negative control of the parent virus (Lane 1) that did not express TTMG-1 antigen and recFPV-MGC3 (Lane 4) did not exhibit any specific bands.

It was confirmed by the Western blotting method that the recombinant fowlpox virus rFPV-B/MG40-S (Lane 5) obtained in Example 8 and the recombinant HVT rHVT/PecMG40KS (Lanes 1 & 2) obtained in Example 9 expressed the TTMG-1 antigen (Figure 2). As the control, rHVT HF-PecHNF (Lane 3) that expresses the HN and F antigen of NDV described in Japanese Unexamined Patent Publication (Kokai) No. 2001-188, the parent fowlpox virus BLEN strain (Lane 4), 40K-S (Lane 5) described in International Patent Publication WO97/36924 were used. Lanes 1 and 2 are HVT having the same structure except that their clones are only different. rFPV-B/MG40-S (Lane 5) that expresses the N-glycosylation site-modified TTMG-1 antigen and rHVT/PecMG40KS (Lanes 1 & 2) expressed about 50 kd TTMG-1 antigen as did rFPV-B/MG40/M11 (Lane 3) in Figure 1. This was consistent with the value of 48.9 kd predicted from the amino acid sequence in which the MDVgB signal was added to the N-glycosylation site-modified TTM-1. On the other hand, 40K-S (lane 6) that expresses the TTMG-1 antigen having a sugar chain is the same virus as in Lane 2 in Figure 1, but the former expressed about 60 kd TTMG-1 antigen. In HF-PecHNF (Lane 3) that expresses NDV, there appears to be a band at about 50 kd, but this was confirmed to be a contamination from Lane 2 in another experiment. In the negative control of the FPV parent strain (Lane 4), no bands were seen. Thus, it was demonstrated that FPV and HVT that express a fusion protein comprised of the N-glycosylation site-modified TTM-1 and the MDVgB signal were not N-glycosylated.

It was confirmed by the western blotting method that the recombinant fowlpox virus rFPV-B/MG-1 (Lanes 2 & 3) obtained in Example 8 expresses the MGC3 antigen (Figure 3). As the negative control, the parent fowlpox virus BLEN strain (Lane 4) was used. According to Yoshida et al. (2000), the size of the fusion protein, expressed by recFPV-MGC3-S, of the MDVgB signal having a N-glycosylation site and MGC3 is 145 kd. The size of the fusion protein of the N-glycosylation site-modified MGC3 antigen that is a gene product of mgc3 of FPV-B/MG-1 obtained in the above Example and the MDVgB signal is 120 kd (Lane 2 & 3). This is consistent with the molecular weight when a sugar chain estimated from the amino acid sequence was not added (not N-glycosylated). This size of 120 kd of the MGC3 antigen is consistent with the fact by Yoshida et al. (2000) that the size of the MGC3 antigen in which the MGC3 antigen of recFPV-MGC3-S was treated with endoglycosidase H and PNGase F and the sugar chain was removed is 120 kd.

Furthermore, for the recombinant fowlpox virus rFPV-B/MG-1 (Lane 1-3) obtained in Example 8, the expressed protein was treated with PNGase F (Lane 1) and endoglycosidase H (Lane 2), and then subjected to Western blotting using Mab 35A6 (Figure 4). As a result, it was demonstrated, that despite the treatment with endoglycosidase H or PNGase F, it was 120 kd and was not N-glycosylated. This also is consistent with the above fact indicated by Yoshida et al. (2000).

### Example 12. Confirmation of the protein expressed from the recombinants (the immunoprecipitation method)

Furthermore, the protein expressed from the recombinant virus was investigated using the immunoprecipitation method.

The CEF monolayer infected with recombinant virus was incubated with a methionine-free growth medium (0.5% FCS). Then, it was incubated in a medium in which [³⁵S]-Met isotope (100 mCi/ml) was added to a Met-free MEM medium (0.5%, FCS) at 37°C for 16-48 hours. After harvesting the cells, they were solubilized by adding the lysing buffer (50 mM Tris (pH 7.5), 150 mM NaCl, 0.5% NP-40, 0.1% aprotinin). After centrifugation to remove the precipitate, anti-TTMG-1 antiserum or Mab 35A6 and Protein G-Agarose (Roche 1243233) were mixed and incubated at 4°C. The precipitate was collected by centrifugation, and washed in the washing Buffer (50 mM Tris (pH 7.5), 150 mM NaCl, 1% Triton X-100, 1% sodium deoxycholate (NaDOC), 0.1% SDS), to which the SDS Sample Buffer was added, and boiled at 100°C for 10 min., and the supernatant was made for the sample. Each sample was subjected to SDS-PAGE, and the gel was dried and exposed to X-ray film to visualize.

For the recombinant fowlpox virus rFPV-B/MG40/M11 (Lane 3) obtained in Example 8, immunoprecipitation was performed using anti-TTMG-1 antiserum (panel A) and Mab 35A6 (panel B) to investigate the expressed protein (Figure 5). The expression of the TTMG-1 antigen can be confirmed in panel A, and the expression of MGC3 antigen in panel B. The recombinant fowlpox virus 40K-S (Lane 2) described in International Patent Publication WO97/36924 is a control in panel B, and the recombinant fowlpox virus recFPV-MGC3 (Lane 4) described in Yoshida et al. (2000) is a control in panel A. The parent fowlpox virus BLEN strain (Lane 1) is a control in both of panels A and B. It was confirmed, in immunoprecipitation using anti-TTMG-1 antiserum, that rFPV-B/MG40/M11 (Lane 3) having the modified TTMG-1 antigen expresses about 50 kd non-N-glycosylated TTMG-1 antigen as in the result of the Western blotting method in Example 11. On the other hand, 40K-S (Lane 2) having the unmodified TTMG-1 antigen expressed about 60 kd N-glycosylated TTMG-1 antigen. It was also confirmed, in immunoprecipitation using Mab 35A6, that rFPV-B/MG40/M11 (Lane 3) having a re-modified mgc3 gene expressed about 120 kd non-N-glycosylated MGC3 antigen as in the result of the Western blotting method in Example 11.

On the other hand, recFPV-MGC3 that expresses an unmodified MGC-3 antigen expressed an about 140 kd N-glycosylated MGC-3 antigen as in the result of the Western blotting method in Example 11.

From the foregoing, as was demonstrated in Examples 10-12, the addition of a N-linked sugar chain (N-glycosylation) can be prevented by deleting a N-glycosylation site.

### Reference Example 3. Mycoplasma challenge test on recombinant FPV-inoculated chickens

The challenge test was performed according to the Japanese Standards for Biological Products of Animals. The method is briefly explained below. Using eggs of white leghorn SPF chickens (chicken species: Line-M, Nippon Institute for Biological Science), 5 weeks after hatch, recombinant FPV was inoculated by wing web stab to 10⁴ pfu per chick. The recombinant FPVs used were two: recombinant FPV described in International Patent Publication WO97/36924, 40K-S, and recFPV-MGC3-S reported by Yoshida et al. (2000). Two weeks after vaccination, chickens were challenged with the virulent MG-R strain. The challenge method was performed according to the Standards for Biological Products of Animals, and the chickens were challenged to the trachea with 4.8 × 10⁴ CFU per chick. On day 14 after the challenge, the chickens were necropsied and dissected to prepare histopathological samples of the trachea, for which the tracheal lesion scores were determined based on the thickness of the mucosal tissue of the trachea and the histopathological findings. As described in International Patent Publication WO97/36924, the criteria of scoring is the same as the Japanese Standards for the Products, and the mean of the bronchial lesion scores of chickens in each group was made the score of the group. The result is shown in Table 3.

**Table 3. Mycoplasma challenge test on recombinant FPV-inoculated chickens - 1**

| | No. of chickens | tracheal lesion score |
|---|---|---|
| Challenge controls | 10 | 2.53 |
| 40K-S | 10 | 1.96 |
| recFPV-MGC3-S | 10 | 2.78 |

The result confirmed that recFPV-MGC3-S had no effect but the recombinant FPV 40K-S had a vaccine effect.

### Example 13. Mycoplasma challenge test on recombinant FPV-inoculated chickens

The challenge test was basically performed according to the USDA-9CFR. The method is briefly explained below. Using eggs of white leghorn SPF chickens (chicken species: Line-M, Nippon Institute for Biological Science), 4 weeks after hatch, recombinant FPV was inoculated by wing web stab to 10⁴ pfu per chick. The recombinant FPVs used were three: recombinant FPV described in International Patent Publication WO97/36924, 40K-S, rFPV-B/MG40-S obtained in Example 8, and rFPV-B/MG40- obtained in Example 8. As the control, the parent virus of the recombinant, the FPV BLEN strain, was also inoculated. The commercially available vaccine of Mycoplasma gallisepticum was inoculated as described in the directions. Three weeks after vaccination, chickens were challenged with virulent MG-R strain by spraying a bacterial solution of 1.0 × 10¹⁰ CCU for 1 minute. 10 days after challenge, the chickens were necropsied, for which the tracheal lesion score was determined by the method of Evans et al. The detailed method of scoring is as follows:

Criteria of scores are as follows;

| Score | findings |
|---|---|
| 0 | normal air sac, clear and thin |
| 1 | only cloudiness or gray areas with slight thickening or flecks of yellowish exudate, involving a very limited area of one or two air sacs |
| 2 | readily visible grayish or yellow exudate, sometimes foamy, with thickening of the air sac, involving one or portions of two air sacs |
| 3 | somewhat more severe exudative, thickened airsacculitis, but mainly more extensive, involving essentially three air-sac regions |
| 4 | severe airsacculitis with considerable exudate and thickening of almost all air-sac regions |

The score for each chicken was determined, and averaged in each group to obtain the mean score. Test of significant difference was performed between the non vaccinated challenge control group and the vaccinated group using the Mann-Whitney U-test.

**Table 4: Mycoplasma challenge test on recombinant FPV-inoculated chickens - 2**

| virus | No. of chickens | Mean score | Significant difference |
|---|---|---|---|
| rFPV-B/MG40-S | 10 | 1.00 | * |
| rFPV-B/MG40- | 10 | 1.88 | * |
| 40K-S | 10 | 1.78 | * |
| Live Mg vaccine | 10 | 2.10 | * |
| Parent FPV | 10 | 3.38 | |
| Challenge controls | 10 | 2.90 | |

| | | | |
|---|---|---|---|
| *: Significant difference with a significance level of 1%. | | | |

Based on the result, the vaccine effect was observed in all of the three recombinant FPVs, and vaccine effects were equal to or better than the live vaccine. Furthermore, it was demonstrated that, among the three, rFPV-B/MG40-S that expresses the non-N-glycosylated TTMG-1 antigen has a higher vaccine effect than rFPV-B/MG40-or 40K-S that expresses the N-glycosylated TTMG-1 antigen.

### Example 14. Mycoplasma challenge test on recombinant FPV-inoculated chickens

The challenge test was performed as in Example 13, and major differences will be described. Using eggs of white leghorn SPF chickens (Line-M, Nippon Institute for Biological Science), 4 weeks after hatch, recombinant FPV was inoculated by wing web stab to 10⁴ pfu per chick. The recombinant FPV used was rFPV-B/MG40/M11. The commercially available vaccine of Mycoplasma gallisepticum was inoculated as described in the directions. Three weeks after vaccination, chickens were challenged with the virulent MG-R strain by spraying a bacterial solution of 1.0 × 10^{10.67} CCU for 1 minute. Ten days after challenge, the chickens were necropsied, for which the air sac lesion score was determined by the method of Evans et al. As in Example 13, the test of significant difference was performed between the vaccinated non challenge control group and the vaccinated group using the Mann-Whitney U-test.

**Table 5: Mycoplasma challenge test on recombinant FPV-inoculated chickens - 3**

| Group | No. of chickens | Mean score | Significant difference |
|---|---|---|---|
| rFPV-B/MG40/M11 | 9 | 1.67 | * |
| Live Mg vaccine | 10 | 1.90 | *2 |
| Challenge controls | 9 | 3.33 | |

| | | | |
|---|---|---|---|
| *: Significant difference with a significance level of 1% *: Significant difference with a significance level of 5% | | | |

Based on the result, rFPV-B/MG40/M11 that expresses the non-N-glycosylated TTMG-1 antigen and the MGC3 antigen exhibited a vaccine effect exceeding the live vaccine.

### Example 15. Mycoplasma challenge test on recombinant FPV-inoculated chickens

The challenge test was performed as in Example 13, and major differences will be described. Using eggs of white leghorn SPF chickens (SPAFAS), 4 weeks after hatching, recombinant FPV was inoculated by wing web stab to 10⁴ TCID₅₀ per chick. The recombinant FPVs used were two: rFPV-B/MG40-S and rFPV-B/MG40/M11. The commercially available vaccine of Mycoplasma gallisepticum was inoculated as described in the directions. Three weeks after vaccination, chickens were challenged with the virulent MG-R strain by spraying a bacterial solution of 1.0 × 10^{8.86} CCU for 1 minute. Ten days after challenge, the chickens were necropsied, for which the air sac lesion score was determined by the method of Evans et al. As in Example 13, the test of significant difference was performed between the vaccinated non challenge control group and the vaccinated group using the Mann-Whitney U-test.

**Table 6: Mycoplasma challenge test on recombinant FPV-inoculated chickens - 4**

| Group | No. of chickens | Mean score | Significant difference |
|---|---|---|---|
| Challenge controls | 20 | 2.15 | |
| Live Mg vaccine | 20 | 0.60 | * |
| rFPV-B/MG40-S | 20 | 1.7 | |
| rFPV-B/MG40/Mll | 20 | 1.15 | * |
| Negative controls | 10 | 0 | |

| | | | |
|---|---|---|---|
| *: Significant difference with a significance level of 1% | | | |

Based on the result, the vaccine effect of two recombinants rFPV-B/MG40-S and rFPV-B/MG40/M11 that express the non-N-glycosylated TTMG-1 antigen was confirmed. Furthermore, rFPV-B/MG40/M11 that expresses the non-N-glycosylated TTMG-1 antigen and the non-N-glycosylated MGC3 antigen exhibited a vaccine effect exceeding rFPV-B/MG40-S that only expresses the non-N-glycosylated TTMG-1 antigen.

### Example 16. Mycoplasma challenge test on recombinant FPV-inoculated chickens

The challenge test was performed as in Example 13, and major differences will be described. Using eggs of white leghorn SPF chickens (Line-M, Nippon Institute for Biological Science), 4 weeks after hatch, recombinant FPV was inoculated by wing web stab to 10⁴ pfu per chick. The recombinant FPVs used were three: rFPV-B/MG-1 to rFPV-B/MG-3 described in Example 8. Three weeks after vaccination, chickens were challenged with the virulent MG-R strain by spraying a bacterial solution of 1.0 × 10^{9.85} CCU for 1 minute. Ten days after the challenge, the chickens were necropsied, for which the air sac lesion score was determined by the method of Evans et al. As in Example 17, the test of significant difference was performed between the vaccinated non-challenge control group and the vaccinated group using the Mann-Whitney U-test.

**Table 7: Mycoplasma challenge test on recombinant FPV-inoculated chickens - 5**

| Group | No. of chickens | Mean score | Significant difference |
|---|---|---|---|
| Challenge controls | 20 | 3.50 | |
| rFPV-B/MG-1 | 20 | 1.35 | * |
| rFPV-B/MG-2 | 19 | 1.74 | * |
| rFPV-B/MG-3 | 18 | 1.26 | * |
| Negative controls | 5 | 0.00 | |

| | | | |
|---|---|---|---|
| *: Significant difference with a significance level of 1% | | | |

Based on the result, vaccine effect was confirmed in all of rFPV-B/MG-1 to rFPV-B/MG-3 that express the non-N-glycosylated MGC3 antigen.

### Example 17. Mycoplasma challenge test on recombinant FPV-inoculated chickens

The challenge test was performed as in Example 13, and major differences will be described. Using eggs of white leghorn SPF chickens (SPAFAS), 8 weeks after hatch, recombinant FPV was inoculated by wing web stab to 10^{3.5} TCID₅₀ pfu per chick. The recombinant FPVs used were three: rFPV-B/MG-1 to rFPV-B/MG-3 obtained in Example 8. The live vaccine of Mycoplasma gallisepticum was inoculated as described in the directions. Three weeks after vaccination, chickens were challenged with the virulent MG-R strain by spraying a bacterial solution of 1.0 × 10^{8.6} CCU for 1 minute. Ten days after challenge, the chickens were necropsied, for which the air sac lesion score was determined by the method of Evans et al. As in Example 14, the test of significant difference was performed between the vaccinated non challenge control group and the vaccinated group using the Mann-Whitney U-test.

**Table 8: Mycoplasma challenge test on recombinant FPV-inoculated chickens - 6**

| Group | No. of chickens | Mean score | Significant difference |
|---|---|---|---|
| Challenge controls | 30 | 1.78 | |
| rFPV-B/MG-1 | 30 | 0.37 | * |
| rFPV-B/MG-2 | 30 | 0.39 | * |
| rFPV-B/MG-3 | 30 | 0.56 | * |
| Live Mg vaccine | 30 | 0.43 | * |
| Negative controls | 5 | 0.00 | |

| | | | |
|---|---|---|---|
| *: Significant difference with a significance level of 1% | | | |

Based on the result, vaccine effect was confirmed in all of rFPV-B/MG-1 to rFPV-B/MG-3 that express the non-N-glycosylated MGC3 antigen.

### Example 18. Mycoplasma challenge test on recombinant FPV- and recombinant HVT-inoculated chickens

The challenge test was performed as in Example 13, and major differences will be described. Using eggs of white leghorn SPF chickens (Line-M, Nippon Institute for Biological Science), 3 days after hatch, recombinant HVT was subcutaneously inoculated to 3 × 10³ PFU per chick, and three weeks after hatch, recombinant FPV was inoculated by wing web stab to 10⁴ TCID₅₀ pfu per chick. The recombinant HVTs used were rHVT/PecMG40KS+2nd and rHVT/Bac40KS-CMVM11 obtained in Example 9. The recombinant FPV used was rFPV/MG-1 obtained in Example 8. The commercially available live vaccine of Mycoplasma gallisepticum was inoculated three weeks after hatch as described in the directions. Seven weeks after hatching, chickens were challenged with the virulent MG-R strain by spraying a bacterial solution of 1.0 × 10^{9.4} CCU for 1 minute. Ten days after the challenge, the chickens were necropsied, for which the air sac lesion score was determined by the method of Evans et al. As in Example 13, the test of significant difference was performed between the vaccinated non challenge control group and the vaccinated group using the Mann-Whitney U-test.

**Table 9: Mycoplasma challenge test on recombinant FPV- and recombinant HVT-inoculated chickens**

| Group | No. of chickens | Mean score | Significant difference |
|---|---|---|---|
| rFPV-B/MG-1 | 15 | 1.20 | *2 |
| rHVT/PeCMG40KS+2nd | 16 | 1.50 | * |
| rHVT/Bac40KS-CMVM11 | 15 | 2.00 | |
| Live Mg vaccine | 14 | 1.79 | * |
| Challenge controls | 12 | 2.75 | |

| | | | |
|---|---|---|---|
| *: Significant difference with a significance level of 1% *2: Significant difference with a significance level of 5% | | | |

Based on the result, vaccine effect was confirmed in the recombinant HVT of the present invention, rHVT/PecMG40KS, that expresses the non-N-glycosylated TTMG-1 antigen and rFPV-B/MG-1 that expresses the non-N-glycosylated MGC3 antigen. Furthermore, the vaccine effect of rHVT/Bac40KS-MVM11 that expresses the non-N-glycosylated TTMG-1 antigen and the non-N-glycosylated MGC3 antigen was confirmed.

### Example 19. Mycoplasma challenge test on recombinant HVT-inoculated chickens

The challenge test was performed as in Example 13, and major differences will be described. Using eggs of white leghorn SPF chickens (Line-M, Nippon Institute for Biological Science), on the day of hatch, recombinant FPV was subcutaneously inoculated to 10³ or 10⁴ TCID₅₀ per chick. The recombinant HVT used was rHVT/PecMG40KS +2nd constructed in Example 9. The commercially available Mycoplasma gallisepticum live vaccine was inoculated three weeks after hatch as described in the directions. Seven weeks after hatch, a MG virulent R strain was challenged by spraying a bacterial solution of 1.0 × 10^{5.59} CCU for 1 minute. Ten days after the challenge, the chickens were necropsied, for which the air sac lesion score was determined by the method of Evans et al. As in Example 13, the test of significant difference was performed between the vaccinated non challenge control group and the vaccinated group using the Mann-Whitney U-test.

**Table 10: Mycoplasma challenge test on recombinant HVT-inoculated chicken**

| Group | Dose log₁₀ /chick | No. of chickens | Mean score | Significant difference |
|---|---|---|---|---|
| rHVT/PecMG40KS+2nd | 4.0 | 8 | 2.00 | * |
| rHVT/PecMG40KS | 3.0 | 7 | 2.57 | * |
| Live Mg vaccine label | | 10 | 2.10 | * |
| Challenge controls | | 12 | 3.50 | |
| Negative controls | | 5 | 0.00 | |

| | | | | |
|---|---|---|---|---|
| *: Significant difference with a significance level of 1% | | | | |

Based on the result, vaccine effect was confirmed in rHVT/PecMG40KS+2nd, a recombinant HVT that expresses the non-N-glycosylated TTMG-1 antigen.

### Reference Cited

US. PATENTE DOCUMENTS
5,180,675 1/1993 Drillien et al. 435/235.1
5,387,519 2/1995 Yanagida et al. 435/235.1
5,489,430 2/1996 Saito et al. 424/190.1
5,766,594 6/1998 Kodama et al. 424/190.1
5,871,742 2/1999 Saitoh et al. 424/199.1

### FOREIGN PATENT DOCUMENTS

WO97/24370 7/1997 WIPO
WO97/36924 10/1997 WIPO
WO99/18215 4/1999 WIPO
2766984 4/1998 Japan.
2001-188 1/2001 Japan.

### OTHER PUBLICATIONS

Andre, S. et al., "Increased immune response elicited by DNA vaccination with a synthetic gp120 sequence with optimized codon usage." J. Virol. 72:1497-1503, 1998.

Davison, A. J. and J. B Moss. "Structure of vaccinia virus early promoters." J. Mol. Biol. 210:749-769, 1989.

Davison, A. J. and B. Moss. "Structure of vaccinia virus late promoters." J. Mol. Biol. 210:771-784, 1989.

Greuel, B. T. et al., "Transcriptional activity of the Rous sarcoma virus long terminal repeat correlates with binding of a factor to an upstream CCAAT box in vitro." Virology 177:33-43, 1990.

Gunning, P. et al., "A human beta-actin expression vector system directs high-level accumulation of antisense transcripts." Proc. Natl. Acad. Sci. U.S.A. 84:4831-4835, 1987.

Kost, T. A. et al., "The nucleotide sequence of the chick cytoplasmic beta-actin gene." Nucleic Acids. Res. 11:8287-8301, 1983.

Morgan, R. W. et al., "Transfection of chicken embryo fibroblasts with Marek's disease virus DNA." Avian Dis. 34:345-351, 1990.

Nakamura Y. et al., "Condon usage tabulated from the international DNA sequence database." Nucleic Acids. Res. 24:214-215, 1996.

Ross L. et al., 16th International Herpes Workshop (1991)

Ross, L. J. N et al., "Construction and properties of a turkey herpesvirus recombinant expressing the Marek's disease virus homologue of glycoprotein B of herpes simplex virus." J. Gen. Virol 74:371-377, 1993.

Sakaguchi, M. et al., "Construction of recombinant Marek's disease virus type I (MDV1) expressing the Escherichia coli lacZ gene as a possible live vaccine vector: the US10 gene of MDVI as a stable insertion site." Vaccine 12:953-957, 1994.

Sondermeijer, P. J. et al., "Avian herpesvirus as a live viral vector for the expression of heterologous antigens." Vaccine 11:349-358, 1993.

Stinski, M. F. and T. J. Roehr. "Activation of the major immediate early gene of human cytomegalovirus by cis-acting elements in the promoter-regulatory sequence and by virus-specific trans-acting components." J. Virol. 55:431-441, 1985.

Yanagida N et al., "Recombinant fowlpox viruses expressing the glycoprotein B homolog and the pp38 gene of Marek's disease virus." Journal of Virology 66:1402-1408, 1992.

Yanagida, N. et al., "Nucleotide and predicted amino acid sequences of Marek's disease virus homologues of herpes simplex virus major tegument proteins." J. Gen. Virol. 74:1837-1845, 1993.

Yoshida, S. et al., "Identification and expression of a Mycoplasma gallisepticum surface antigen recognized by a monoclonal antibody capable of inhibiting both growth and metabolism" Infect. Immun., vol. 68, pp. 3186-3192, 2000.

Weir, J. P. and B. Moss. "Regulation of expression and nucleotide sequence of a late vaccinia virus gene." J. Virol. 51:662-669, 1984.

| Sequence listing | Free text |
|---|---|
| Sequence No. 1 | TTM-1 gene (behind EcoRI) |
| Sequence No. 2 | mgc3 gene |
| Sequence No. 3 | HVT-UL44-46 insertion site |
| Sequence No. 4 | FPV-29 insertion site |
| Sequence No. 5 | MDVgB signal (amino acid) |
| Sequence No. 6 | modified MDVgB signal (amino acid) |
| Sequence No. 7 | modified MDVgB signal (gene) |
| Sequence No. 8 | Rabies virus glycoprotein G (gG) signal (amino acid) |
| Sequence No. 9 | synthetic DNA-1: 1BN for Rabies gG signal |
| Sequence No. 10 | synthetic DNA-2: 1BC for Rabies gG signal |
| Sequence No. 11 | modified Rabies virus glycoprotein G signal |
| Sequence No. 12 | TTM-1 portion of pNZ40K-S (amino acid) |
| Sequence No. 13 | synthetic DNA (primer): 40KG-1 (23 mer) |
| Sequence No. 14 | synthetic DNA (primer): 40KG-2R (22 mer) |
| Sequence No. 15 | synthetic DNA (primer): 40KG-2 (23 mer) |
| Sequence No. 16 | synthetic DNA (primer): 40KG-3R (21 mer) |
| Sequence No. 17 | synthetic DNA (primer): 40KG-3A (36 mer) |
| Sequence No. 18 | synthetic DNA (primer): 40KG-4RA (34 mer) |
| Sequence No. 19 | synthetic DNA (primer): 40KG-4 (24 mer) |
| Sequence No. 20 | synthetic DNA (primer): 40KG-5R (28 mer) |
| Sequence No. 21 | synthetic DNA (primer): 40KG-5 (27 mer) |
| Sequence No. 22 | synthetic DNA (primer): 40KG-6R (37 mer) |
| Sequence No. 23 | TTM-1 portion of modified pNZ40K-S (downstream of BglI) (amino acid) |
| Sequence No. 24 | TTM-1 portion of the modified pNZ40K-S (downstream of BglI) (gene) |
| Sequence No. 25 | MGC3 protein encoded by the mgc3 gene (amino acid) |
| Sequence No. 26 | synthetic DNA (primer): M11-B (26 mer) |
| Sequence No. 27 | synthetic DNA (primer): M11-2R (20 mer) |
| Sequence No. 28 | synthetic DNA (primer): M11-2 (20 mer) |
| Sequence No. 29 | synthetic DNA (primer): M11-3R (20 mer) |
| Sequence No. 30 | synthetic DNA (primer): M11-3 (20 mer) |
| Sequence No. 31 | synthetic DNA (primer): M11-4RB (30 mer) |
| Sequence No. 32 | synthetic DNA (primer): M11-4B (30 mer) |
| Sequence No. 33 | synthetic DNA (primer): M11-5R (20 mer) |
| Sequence No. 34 | synthetic DNA (primer): M11-5A (30 mer) |
| Sequence No. 35 | synthetic DNA (primer): M11-7RA (30 mer) |
| Sequence No. 36 | synthetic DNA (primer): M11-7 (20 mer) |
| Sequence No. 37 | synthetic DNA (primer): M11-KR (20 mer) |
| Sequence No. 38 | synthetic DNA (primer): M11-K (20 mer) |
| Sequence No. 39 | synthetic DNA (primer): M11-8R (30 mer) |
| Sequence No. 40 | synthetic DNA (primer): M11-8 (30 mer) |
| Sequence No. 41 | synthetic DNA (primer): M11-10R (25 mer) |
| Sequence No. 42 | synthetic DNA (primer): M11-10 (25 mer) |
| Sequence No. 43 | synthetic DNA (primer): M11-12RA (30 mer) |
| Sequence No. 44 | synthetic DNA (primer): M11-12A (30 mer) |
| Sequence No. 45 | synthetic DNA (primer): M11-XR (20 mer) |
| Sequence No. 46 | synthetic DNA (primer): M11-XA (30 mer) |
| Sequence No. 47 | synthetic DNA (primer): M11-13RA (30 mer) |
| Sequence No. 48 | synthetic DNA (primer): M11-13A (30 mer) |
| Sequence No. 49 | synthetic DNA (primer): M11-14RA (30 mer) |
| Sequence No. 50 | synthetic DNA (primer): M11-14A (30 mer) |
| Sequence No. 51 | synthetic DNA (primer): M11-15RA (30 mer) |
| Sequence No. 52 | synthetic DNA (primer): M11-15A (30 mer) |
| Sequence No. 53 | synthetic DNA (primer): M11-16RA (30 mer) |
| Sequence No. 54 | synthetic DNA (primer): M11-16A (30 mer) |
| Sequence No. 55 | synthetic DNA (primer): M11-GTR (30 mer) |
| Sequence No. 56 | synthetic DNA (linker): H'-S-H-S-P-S1 (36 mer) |
| Sequence No. 57 | synthetic DNA (linker): H'-S-H-S-P-S2 (36 mer) |
| Sequence No. 58 | synthetic DNA (linker): S-B-E1 (35 mer) |
| Sequence No. 59 | synthetic DNA (linker): S-B-E2 (35 mer) |
| Sequence No. 60 | synthetic DNA (primer): M11-Sfi (30 mer) |
| Sequence No. 61 | synthetic DNA (primer): M11-5RB (30 mer) |
| Sequence No. 62 | synthetic DNA (primer): M11-5C (30 mer) |
| Sequence No. 53 | synthetic DNA (primer): M11-KRA (30 mer) |
| Sequence No. 64 | synthetic DNA (primer): pCMV-1 (30 mer) |
| Sequence No. 65 | synthetic DNA (primer): pPec1R (30 mer) |
| Sequence No. 66 | synthetic DNA (primer): pCMV-01 (30 mer) |
| Sequence No. 67 | synthetic DNA (primer): pCMV-R1 (30 mer) |
| Sequence No. 68 | synthetic DNA (primer): pGHP-F (29 mer) |
| Sequence No. 69 | synthetic DNA (primer): pGHP-R (30 mer) |
| Sequence No. 70 | synthetic DNA (linker): Linker 1 (23 mer) |
| Sequence No. 71 | synthetic DNA (linker): Linker 2 (23 mer) |
| Sequence No. 72 | synthetic DNA (linker): Linker 3 (16 mer) |
| Sequence No. 73 | synthetic DNA (linker): Linker 4 (12 mer) |
| Sequence No. 74 | synthetic DNA (primer): PolyA-SfiF2 (30 mer) |
| Sequence No. 75 | synthetic DNA (primer): PolyA-SanlKpn (30 mer) |
| Sequence No. 76 | synthetic DNA (primer): 40KS-B (22 mer) |
| Sequence No. 77 | synthetic DNA (primer): pMG40K-1 (30 mer) |
| Sequence No. 78 | modified MGC3 antigen (M11-BTR) (amino acid) |
| Sequence No. 79 | modified mgc3 gene (M11-BTR) (DNA) |

## Claims

1. A recombinant herpesvirus that has integrated therein a DNA encoding TTM-1 and comprising a DNA sequence as shown in SEQ ID NO: 1, wherein at least one of the DNA regions encoding NXB (N is asparagine, X is any amino acid other then proline, and B is serine or threonine) has been modified so that no N-glycosylation occurs during expression in a eukaryotic cell.

2. The recombinant herpesvirus according to claim 1, wherein the modification in the DNA sequence as shown in SEQ ID NO: 1 is at least one of the following:
(i) the modification of the DNA sequence encoding asparagine (N) to a DNA sequence encoding an amino acid other than asparagine;
(ii) the modification of DNA sequence encoding any amino acid (X) other than proline to a DNA sequence encoding proline; or
(iii) the modification of the DNA sequence encoding serine or threonine (B) to a DNA sequence encoding an amino acid other than serine or threonine.

3. The recombinant herpesvirus according to claim 1 or 2, wherein a DNA encoding a signal sequence has been ligated to the N-terminal end of the DNA encoding TTM-1.

4. The recombinant herpesvirus according to claim 3, wherein the DNA sequence encoding the signal sequence has been modified to that no N-glycosylation occurs during the expression in a eukaryotic cell.

5. The recombinant herpesvirus according to claim 4, wherein the signal sequence is a signal sequence derived from the gB of Marek's disease virus or a signal sequence derived from the gB of Rabies virus.

6. The recombinant herpesvirus according to claim 4 or 5, wherein the virus is a Marek's disease virus type I, type II or type III.

7. A vaccine comprising a recombinant herpesvirus according to any one of claims 1 to 6.

8. A DNA molecule derived from a prokaryotic cell in which at least one of the DNA regions encoding NXB (N is asparagine, X is any amino acid other than proline, and B is serine or threonine) has been modified so that no N-glycosylation occurs during the expression in a eukaryotic cell.

9. The DNA molecule according to claim 8, wherein said alteration that attempts to prevent N-glycosylation is at least one of the following:
(1) the alteration of the DNA sequence encoding asparagine (N) to a DNA sequence encoding an amino acid other than asparagine;
(2) the alteration of the DNA sequence encoding any amino acid (X) other than proline to a DNA sequence encoding proline; and
(3) the alteration of the DNA sequence encoding serine or threonine (B) to a DNA sequence encoding an amino acid other than serine or threonine.

10. The modified DNA molecule according to claim 8, wherein said DNA molecule derived from a prokaryotic cell is a DNA encoding an antigen protein.

11. The modified DNA molecule according to claim 8, wherein said prokaryotic cell is Mycoplasma.

12. The modified DNA molecule according to claim 8, wherein said DNA molecule derived from a prokaryotic cell is a DNA derived from Mycoplasma having the DNA sequence according to claim 8 or 9.

13. A fused DNA molecule, wherein a DNA encoding a signal sequence has been ligated to the N-terminal end of the modified DNA molecule according to claim 8 so that it may be expressed as a fusion protein.

14. The fused DNA molecule according to claim 13, wherein at least one of the DNA regions of DNA encoding said signal sequence in which said signal sequence-encoding DNA comprises DNA regions encoding NXB (N is asparagine, X is any amino acid other than proline, and B is serine or threonine) has been modified so that no N-glycosylation occurs during the expression in the eukaryotic cell.

15. The fused DNA molecule according to claim 13, wherein said signal sequence is a signal sequence derived from the gB of Marek's disease virus or a signal sequence derived from the gG of Rabies virus.

16. The fused DNA molecule according to claim 13, wherein said DNA molecule derived from a prokaryotic cell has a DNA sequence described in SEQ ID NO: 1 or 2 derived from Mycoplasma, and said signal sequence is a signal sequence derived from the gB of Marek's disease virus or a signal sequence derived from the gG of Rabies virus.

17. A recombinant virus that has integrated therein
(1) a DNA molecule derived from a prokaryotic cell in which at least one of the DNA regions encoding NXB (N is asparagine, X is any amino acid other than proline, and B is serine or threonine) has been modified so that no N-glycosylation occurs during the expression in a eukaryotic cell, or
(2) a fused DNA molecule in which a DNA encoding a signal sequence is ligated to the N-terminal end of said modified DNA molecule so that it may be expressed as a fusion protein.

18. The recombinant virus according to claim 17, wherein said alteration that attempts to prevent N-glycosylation is at least one of the following:
(1) the alteration of the DNA sequence encoding asparagine (N) to a DNA sequence encoding an amino acid other than asparagine;
(2) the alteration of the DNA sequence encoding any amino acid (X) other than proline to a DNA sequence encoding proline; and
(3) the alteration of the DNA sequence encoding serine or threonine (B) to a DNA sequence encoding an amino acid other than serine or threonine.

19. The recombinant virus according to claim 17, wherein said DNA molecule derived from a prokaryotic cell is a DNA molecule derived from Mycoplasma having the DNA sequence according to claim 8 or 9.

20. A recombinant virus that has integrated therein a fused DNA molecule, wherein a DNA encoding a signal sequence that has been modified so that no N-glycosylation occurs during the expression in a eukaryotic cell has been ligated to the N-terminal end of a DNA molecule derived from a prokaryotic cell in which at least one of the DNA regions encoding NXB (N is asparagine, X is any amino acid other than proline, and B is serine or threonine) has been modified so that no N-glycosylation occurs during the expression in a eukaryotic cell, so that it may be expressed as a fusion protein.

21. The recombinant virus according to claim 20, wherein said signal sequence is a signal sequence derived from the gB gene of Marek's disease virus or a signal sequence derived from the gG gene of Rabies virus.

22. The recombinant virus according to claim 17 or 13, wherein said virus is a poxvirus or a herpesvirus.

23. The recombinant virus according to claim 17 or 13, wherein said virus is a virus that infects avians.

24. The recombinant virus according to claim 17 or 13, wherein said virus is an avipoxvirus.

25. The recombinant virus according to claim 17 or 13, wherein said virus is a Marek's disease virus type I, type II, or type III.

26. A method of producing an modified protein or a fusion protein comprising the same, said method comprising using:
(1) a recombinant virus that has integrated therein a DNA molecule derived from a prokaryotic cell in which at least one of the DNA regions encoding NXB (N is asparagine, X is any amino acid other than proline, and B is serine or threonine) has been modified so that no N-glycosylation occurs during the expression in a eukaryotic cell, or
(2) a recombinant virus that has integrated therein a fused DNA molecule in which a DNA encoding a signal sequence has been ligated to the N-terminal end of said modified DNA molecule so that it may be expressed as a fusion protein,
to express a protein encoded by said modified DNA molecule or said fused DNA molecule in a eukaryotic cell.

27. A vaccine comprising the recombinant virus according to claim 17 or 20.


SEQUENCE LISTING

<110> Nippon Zeon Co., Ltd.,
<120> Modified DNA molecules, Recombinants and uses thereof
<130> HMJ04258EP
<140>
<141>
<160> 79
<170> PatentIn Ver. 2.1
<210> 1
<211> 1152
<212> DNA
<213> Mycoplasma gallisepticum
<223> TTM-1 gene (after EcoRI)
<400> 1
gaattctgta tgtctattac taaaaaagat gcaaacccaa ataatggcca aacccaatta 60
gaagcagcgc gaatggagtt aacagatcta atcaatgcta aagcgatgac attagcttca 120
ctacaagact atgccaagat tgaagctagt ttatcatctg cttatagtga agctgaaaca 180
gttaacaata accttaatgc aacattagaa caactaaaaa tggctaaaac taatttagaa 240
tcagccatca accaagctaa tacggataaa acgacttttg ataatgaaca cccaaattta 300
gttgaagcat acaaagcact aaaaaccact ttagaacaac gtgctactaa ccttgaaggt 360
ttgtcatcaa ctgcttataa tcaaattcgc aataatttag tggatctata caataaagct 420
agtagtttaa taactaaaac actagatcca ctaaatgggg gaacgctttt agattctaat 480
gagattacta cagttaatcg gaatattaat aatacgttat caactattaa tgaacaaaag 540
actaatgctg atgcattatc taatagtttt attaaaaaag tgattcaaaa taatgaacaa 600
agttttgtag ggacttttac aaacgctaat gttcaacctt caaactacag ttttgttgct 660
tttagtgctg atgtaacacc cgtcaattat aaatatgcaa gaaggaccgt ttggaatggt 720
gatgaacctt caagtagaat tcttgcaaac acgaatagta tcacagatgt ttcttggatt 780
tatagtttag ctggaacaaa cacgaagtac caatttagtt ttagcaacta tggtccatca 840
actggttatt tatatttccc ttataagttg gttaaagcag ctgatgctaa taacgttgga 900
ttacaataca aattaaataa tggaaatgtt caacaagttg agtttgccac ttcaactagt 960
gcaaataata ctacagctaa tccaactcca gcagttgatg agattaaagt tgctaaaatc 1020
gttttatcag gtttaagatt tggccaaaac acaatcgaat taagtgttcc aacgggtgaa 1080
ggaaatatga ataaagttgc gccaatgatt ggcaacattt atcttagctc aaatgaaaat 1140
aatgctgata ag 1152
<210> 2
<211> 3189
<212> DNA
<213> Mycoplasma gallisepticum
<223> mgc3 gene

<400> 2
atgaatattt ctaaaaaact taaaagttat acattgatag gtggattagc tgtatttgga 60
gctcttggtt ctgcaagctt tggctttaag caatcagata agagtaacga taacacgcaa 120
ttagttaatc aagcaagaac gctagatgct aattctgtta gacttgcagg tcttggacaa 180
aatggttcgt tgttcaatac agttcttaga gatgttgatg ataactttat aacagcagct 240
aatggaacaa ttatcaaatt agatagtttt actaaaccat tatatggttt agatctaagt 300
gatgattgtg gtggatacaa agtaaaacaa atagtttcag attacacaac tagcagaaat 360
agatttgatc aaagacaaac aagagcatat tatgctctgt tggttaatga tgaagctaac 420
gttcatttaa aaagaattaa tactaactca aatagaattg gtaatagaaa caacaattct 480
aagtttgtaa ttggtggtgt tgataatcca gctcacgtaa ttagatttac tgatgatggg 540
actaaattta attttacaaa ccaaactcaa ggtgaaattg ttaatgactt cattttagat 600
gcgccaatct tacctaaaga tttacaccca gattggtata acttatacat tcaaagaaag 660
atcttaccaa atgacgtcaa cactgcagtt gttccttggc cagtaggtag agttagtgga 720
acaaatgctg atgatgggat gtttgattgt gggaatggtc aaataactaa tacagatcct 780
attgctcaaa ctaaaaccac tactgataat caaaatcctt caacttttaa ttcaggagca 840
atgcctggtg caaacaatag atacgattct caattgaatg tcaagcatag aattaaaaca 900
tctttccaat tagatgaaaa atttgtttat ccagaatgga ctggttctga agagaataaa 960
aatattacaa gattagctac tggaagtttg ccaagcaacg aaagatattg gattcttgac 1020
atacccggga ctccacaagt tactttaaaa gaagattcag ttaacgtatt ttcaagacta 1080
tacttaaact cagttaattc tttatcattc attggtgata gtatttatat ttttggtacc 1140
tctgaattac catcattatg gtactattca ttcccaacta gattatctga tctaaccgct 1200
ttgaatcaag ttaaaacaga tgatattgaa gcttcaagca ctgataacgg tacaacaaca 1260
aacggaacaa cgacaacagc tgatacatct agtggttcaa caggtgctgg aacaggaaat 1320
actactaaca cttctcaaac agtttctaat cctactttaa atacttatcg tagttttgga 1380
attgatagta aaccaacttc tgcaaacaaa atagatgaaa ctaattgggc agatcctaac 1440
gttattgaag caagaatata tgctgaatac agattaggta ttcaaaatga aattccaata 1500
actaatgcag gaaactttat ccgaaacaca attggtggtg ttggttttac ttcaacaggt 1560
tcaagagtag ttttaagagc ttcttataac ggtgatcaac gtccaactgg aaacttccaa 1620
cctttcttat acgtatttgg ttatttagga taccaacaaa ctagaacagg aactttctgg 1680
tacggaacat ataagctttt aaacaacagc ccttacgacg tattagattc tccaagagta 1740
ggtactgaaa ccaatcaatt tagaagaact tcattaacat accctgttat gggtggatat 1800
ctaactgaag aaggtgctag aagtttctct aatactccat atataagagc acaaggtgac 1860
acaccagaaa gccgaagcat cttccaatct ggctattctg ataatactta tgagtacatt 1920
caatcagttt taggatttga tggaattaga aataacttaa atgttggggt taaagcatca 1980
agcttcttaa actcaaatag accaaatcca aacggtctag aaatgattgc tgcaacaaca 2040
tacttaagat cacaaattgg attagctaga acatctggat taccaaacca acaaccattc 2100
ggaacaactc accaagttat ttcagtatca cctggtgatc agttctcatc aattaagaat 2160
attagaacaa tcttccctgg taaccagtta tggtacttct tattcacaaa tgaaaataat 2220
aaatctagtg tttatacatt aagattagct gactcaagta accctgatgc gtcaagctca 2280
ttcagtccaa caagtttaat tgacgttaat gaaattggtg taatcttacc tttattagac 2340
aattcattct atacagtaaa tgctgctggt aatgttgcat tgttctcatc aaaccctggt 2400
tctcctggat catatactgc tgtaaataca tttaatcaga acttatctga tattgctttt 2460

gaaggttctg gtgctaagta tacatctgat ttctggggaa caatccaatt caaacccgat 2520
gagtacttaa ttcaaaatgg gttcactagt caagtggcta gaaacttcgt tacaaaccaa 2580
agcttcttaa acagtttagt tgacttcact cctgctaatg ctggtactaa ctaccgtgta 2640
gtggttgatc ctgatggtaa tttaacaaac caaaacctac ctctaaaagt tcagatccaa 2700
tacttagatg gtaagtatta tgatgctaaa ttaaagaaca ataatttagt aacattctct 2760
tataacaact ttggcgcctt accttcatgg gtagtgccta cagcaattgg tagtacatta 2820
ggtattcttg caattatgat catcttagga ttagctatcg gtattccttt aagagctcaa 2880
agaaaattac aagacaaagg gttcaaaaca acattcaaaa aagttgatac cttgactgct 2940
gctgttggtt cagtttacaa gaagattatt acccaaactg ctaacgttaa gaaaaaacct 3000
gctgctttag gtgctggtaa atctggtgat aagaaacctg ctgctgctgc taaacctgct 3060
gctccagcta aaccatctgc accaaaagct agctcaccag ctaaaccaac tgggcctaaa 3120
tctggtgcgc ctacaaaacc aactgctcct aagccagctg ctccaaaacc aaccgctccc 3180
aaagaataa 3189
<210> 3
<211> 2845
<212> DNA
<213> Herpesvirus Turkey
<223> HVT-UL44-46 insert
<400> 3
catggaagaa atttcctcct taccatcaga caaatggagt tcgacatcat tgctttgtcg 60
aattataggc aatgtcatcg cccccttact acaaaccagc gtattatggt cgcaaacccg 120
cctggtggac tttgatggtg atcggaactc acgtgccata ctagcagtgg tttcgttggt 180
ttcacttctc aaccatcatt gtcagtacct aattaattta actcagaggg gatatgtgat 240
atggttggac ggtaatacgg aaaatactat attaaaagag gccctgttgc agcaagacca 300
atttgaacat cagcttggga atttgtttcc gagcatgttg cccgtgtcat ggggggctct 360
cgagtgcagc ataaatactt ggttcggccg cgctgcaacc gatgatgtta aactgtctaa 420
gaacgccctg gctctactgg ggataaaatt actgccaaag cgaataagcg atgttaatgc 480
acggcttatg aaatctattc ggaagcaaga taaaaactcg aacaagtcgg gaagtacgcc 540
cacacatcaa ttaggcgatc gtgtcgaatg tggctgtggc gatacggttc ctggtttgac 600
tgaatttgcg ggggcggggt cgagttctca atgtgcgaac atcggaaacg gtctgactgg 660
tgcaattcgt aagaccgatg ggtctggggg gccttctttg gcgagcaatg accctccagt 720
ttcggaatct ggcagggtgt ctactccgac aaccaaatac tttcatgaaa aaacaatgcg 780
tggagatata ccagtgtcca aggtagaaca tattttacgt aggctgaaag tgtccagcga 840
ataatatttt acacgcgatg cgcggtctgt ataatacatt atatgaaata aagatccata 900
ttatgtttat aatgtctatg gtgtgattgg ataaataaaa cacagtaacc gttagaggtg 960
cgtttttatt tactcatcgc attagagagg acagaggcgt agacttcata ataatcgcgt 1020
cgttctaaat gaagatacac gcgggcatct ctaatgatgg aagacatggt acttagaggc 1080
ctgggtcgct gacatataac ggtacatgtt acaggacagt ctgccgacac gggtcctccc 1140
atggtaggtc gtatacacgt ggttttgctg tcccttaacc ggaagacttc cccatatgct 1200
gcatttggca caccgaatgg ggcgatagct tcaaccagaa ctttggcgtt tgcgaagtca 1260
ataagtttgc tgttatgccg agcacatgta tccaacgcct caagatcagt tgcatttttc 1320

ccggccacgc gcatgcaact ataacccaaa ccaatccatt catcgggaca tgttcccgat 1380
tccatggcca tgtatgaaac agtgaatagt gatcccaaca atactgccgc tacgagaacg 1440
aatatggtaa ataccattcc tatcgtacac ccacaacaga tcgatcgcca agtcgtacgt 1500
ggatgtcgtt gctctctatc tgtttccgtg ccgctatcca tcattcggag acgtccacga 1560
accacaacga gatcgcgatc atcttcaggg gtgggcgaca tcattcttat atgatatacc 1620
ccagcgattt tgtgattgtg tatcactact gagtaactat ataggaaatc tactcagcga 1680
ttgtacggta tccgtatcac attcgatgcg taagattatt accgacgttt tggccgtgag 1740
cttgatattc ggagacttgc gatttttaat gtagcggcac cgtatacgat taacgtgcgc 1800
cacatgatgt acgtcccgat gttttaaata ccgcgtttgg agtaaaaata acataatggg 1860
cattttggcc gctgcgtgat accaaaaata cgtattttga catcacgcgg actgaacatg 1920
agtcatctac gaaacaatgc gatggtgcgg attacaatac cattttattt attgtgaatc 1980
gaaacagaag aatgcgatcg gttataacac ttaataattt ttatatcaca tacgatggtt 2040
aatcttaatt ccgccccggt aggtaaaagc atagggctgt gataatgata ccaataccta 2100
aaaacaaggc cagtcctaga acggccgtaa ttgttacaat catcggcatt cctcttgccg 2160
acggcgttgc atcataatat tcggaatttt gaaatctatc cacgtcgaac ggatatccaa 2220
ttagtctgca cgtatacttc gttttcgccc agttgtcctg gactggaatc cgactggcga 2280
gatttctata acgatcgatg gtcctgcaga gacctgtaac cacagtatca taagttgtgt 2340
taggctgggg ttcatcatga actaaccacc tcacagtaat cccagaggga acacattcta 2400
tagtacatat tgcataccca tctttaaatg ccagagagat acgggggtga taatataccg 2460
tcggtacagc tgtagcattc gatgtgctta tcatatcgcc ttgcctccac gctaccaagc 2520
aggaaacctt tggaggagat tcgagtccag agtttccgag ggttattgtg gatactagtg 2580
tggccccgcg gccagattcg aaccaccaaa aactcccgtc acggtccttg cggggtgtgg 2640
caatgtttcc gttacgtctc caagatacgt agacagatcc cgggggataa aaatgtctaa 2700
cgatgcaaga tgctttgtag ttttctccgc tgagaactgg aggggccaat acatccacgg 2760
atgcgggtcg ttggcacaca cgcatgtaaa tatgtttttc taccaatggg cgattaaatc 2820
tatcacgcag tatcacttga aagct 2845
<210> 4
<211> 3031
<212> DNA
<213> Fowlpox virus
<223>FPV-29 position insert
<400> 4
actagtatca tcatacctaa atacaagaac atattcgttt cctaattttt ctccaataga 60
atttaataga tcgtaagact tatccatacc gagtatttct acgacctttg atttatcgaa 120
gaatgtagct atatttattc taacgatttc atatttacta gctatctttt tattcgaaag 180
tatttcgttt gaaaatttac ataacgggca atttggttta ccaaacaata taatagttcc 240
tttcatttaa tgcgatcaaa aataatggaa ctacatagta ttttatttgt aattaagata 300
gttttatggt tttaactata atgtaaaaat aaggcataaa ttatactttt ttctattatg 360
tacgttacac atatacttgt gttactaaca tgttttcagt actatcatgc agctgtatta 420
tacgataccc agacatctag taatgataat actactgttt atttatacga acccgcttgt 480
gaattagtaa ctacatgggt agatattaga gaagtaagcc ctgttaatgg aactggtgtc 540

agtaaaatag aatttttggg cagatattct gagaaaggag atggatacac acaatacatt 600
caaatgatta gctgcatggg attccctaat tctacatctc cttgtaaggg cattgatgag 660
tcaagatggg aatcttactg ttctacggtc acagaaatta aaaaaataat gtatcttggt 720
gaaaataatg ctgtaatgac taaacatgta tcagttcctg taaattgtgt atgcatggtt 780
aaaagaaaat gaataaacca gtttttttcct tataataaaa aatgtaaaaa caatactatt 840
ctgaatgaaa gtattacaat atggagtttg tacctaacac cgttaaacat atagatgaga 900
acttttgctt tatcaactat gcgaacatag aagtaatcat gttaaaatac aacggatata 960
taaatgctac taaaatatgt gatcttggaa ataagaactt taggcagtgg tgtagactag 1020
agtcatctaa aaaactcata aaaacattga actataaaaa cggtatctat aataaagccg 1080
ttttagaaat cggattagca tcaaatagtg cctataaata cgaacttgta ggaacatatg 1140
tgcatatcga tctcgtgcct catattatct gttgggtttt tcctagtatt gcacttaatt 1200
tttccaagat acttaactcc tatctttcta actcctattg cacgaggctt aaaaaaggtt 1260
ctgataatga agatcagata cgaagcggct tcttctctga gggtataagt aaaatactat 1320
acgacattca tgataatgag atattaaaat taaagaaaaa tacaaaaaaa ttggaagaaa 1380
agtacgaaaa aaccaatagt ttaataaacc aaaaaataag taacttagaa gttgccgtta 1440
aaggtttatc cataaaataa gatatgatag acacttcaat atatcttgct atattatctt 1500
cttttatagc agttatcatg aataatatta gtttgccatt aactgctatc tttattatta 1560
ctatgtatgt tattcatatt cgattggcga ttaatataat gtttctctat atattatcat 1620
ggataataga tattaatatc ttaataataa tatttttagg aattatattt atgcgaaaaa 1680
caggtaaata tctaaataat tatattaggt cacgtaaatc aagtaggaag atcaccaggt 1740
atacagttct attattagat aaggaaaaac ctaattagat aaagattaca tattttcttt 1800
aattaaatca cttcaatata gaaaaagaat taatatataa ttttatatta gagaaaatta 1860
ttatcccaaa acagaaaatg atataaacac gacgtcaaag aaataaaatt gtaggacatg 1920
atgatcttag aaacgctgta gtagttacta catacgcaga ttaattgata aataaatacc 1980
atacatactt agtaggatag atatcgtgtc cagaagaaat atctaaatta acatgatgta 2040
tagaactatt tttattctat gtttgataat acccgtactc ttgaatccga tagactatgg 2100
cgatgaagga gattacagta cagttagtta tgaggaagat gatagtgacg aaactatttt 2160
tctagaccta gaaggatatc ttccggaaaa cgcaagaaaa aattccgacg gtgtgcgaga 2220
atgctgtcct gtaacacgcg aagattgtac ctacgttaat attactagac tgcctgaaga 2280
tataaatact actagcttaa tatgtgtagg gtgtggtagg aaaggttcgt cagcgagaat 2340
gttttgggcc ggtcccggtg gtaaacccat tagcgaaatg gaatcggtaa ttcataaaag 2400
tcctgtaaca attccgttac aaaactctac attttttatt agagaactat tagtggataa 2460
taaacataat ggtaaggaat ttatgtgttt actagtggaa tcaaatacta cagtgtaccg 2520
tcaattatta ctgagacata ttctattact taattagtaa gttcatatat aaataagaat 2580
ggcataccat acgcgcccct gtaattttag aaggctaatc tttatatgtg tattaggtat 2640
agtatgcaag ggatcgttac tccaatttac taatccttat cttataaacg ataaattaga 2700
tacatctaaa gttgttttta gtactcgtac tccgaatata gaacatcgtg gtaaacgaag 2760
catagatgaa ttattgtctg taggtaatac cagtgaagga atatacttat cttgtgaaag 2820
tagttctaca tgggtcgcta acaaaactac agtctttgat cacagaggta ataaactaga 2880
actattggat caaatagtac ataataaaca agtttactat cagtacctat tcgaaaccaa 2940
gtgtaaggaa tatccggcta ttagtggctg tttaggaata gatacacgat tttggtcctc 3000
ttattgttct acgtctcatt cttttgttaa c 3031

<210> 5
<211> 62
<212> PRT
<213> Marek's disease gammmaherpesvirus
<223> MDVgB signal
<400> 5
Met His Tyr Phe Arg Arg Asn Cys Ile Phe Phe Leu Ile Val Ile Leu
1               5                   10                  15
Tyr Gly Thr Asn Ser Ser Pro Ser Thr Gln Asn Val Thr Ser Arg Glu
            20                  25                  30
Val Val Ser Ser Val Gln Leu Ser Glu Glu Glu Ser Thr Phe Tyr Leu
        35                  40                  45
Cys Pro Pro Pro Val Gly Ser Thr Val Ile Arg Leu Glu Phe
    50                  55                  60
<210> 6
<211> 62
<212> PRT
<213> Marek's disease gammaherpesvirus
<223> Modified VgB signal
<400> 6
Met His Tyr Phe Arg Arg Asn Cys Ile Phe Phe Leu Ile Val Ile Leu
1               5                   10                  15
Tyr Gly Thr Gln Ser Ser Pro Ser Thr Gln Gln Val Thr Ser Arg Glu
            20                  25                  30
Val Val Ser Ser Val Gln Leu Ser Glu Glu Glu Ser Thr Phe Tyr Leu
        35                  40                  45
Cys Pro Pro Pro Val Gly Ser Thr Val Ile Arg Leu Glu Phe
    50                  55                  60
<210> 7
<211> 195
<212> DNA
<213> Rabies virus
<223> Modified VgB signal
<400> 7
ggatccaaaa tgcactactt ccgccgcaac tgcatcttct tcctgatcgt gatcctgtac    60
ggcacccaga gcagccccag cacccagcag gtgaccagcc gcgaggtggt gagcagcgtg   120
cagctgagcg aggaggagag caccttctac ctgtgccccc cccccgtggg cagcaccgtg   180
atccgcctgg aattc                                                    195
<210> 8
<211> 23

<212> PRT
<213> Rabies virus
<223> G glycoprotein signal
<400> 8
Met Val Pro Gln Val Leu Leu Phe Ala Pro Leu Leu Val Phe Pro Leu
1               5                   10                  15
Cys Phe Gly Lys Phe Pro Glu
            20
<210> 9
<211> 75
<212> DNA
<213> Artificial Sequence
<220>
<223> Description of Artificial Sequence:1BN for rabies
      gG signal
<400> 9
gatccaaaat ggtgccccag gtgctgctgt tcgcccccct gctggtgttc cccctgtgct    60
tcggcaagtt ccccg                                                    75
<210> 10
<211> 75
<212> DNA
<213> Artificial Sequence
<220>
<223> Description of Artificial Sequence:1BC for rabies
      gG signal
<400> 10
aattcgggga acttgccgaa gcacaggggg aacaccagca ggggggcgaa cagcagcacc    60
tggggcacca ttttg                                                    75
<210> 11
<211> 79
<212> DNA
<213> Artificial Sequence
<220>
<223> Description of Artificial Sequence:Modified rabies
      gG signal
<400> 11
gatccaaaat ggtgccccag gtgctgctgt tcgcccccct gctggtgttc cccctgtgct    60
tcggcaagtt ccccgaatt                                                79
<210> 12

<211> 384
<212> PRT
<213> Mycoplasma gallisepticum
<223> TTM-1 portion of pNZ40K-S
<400> 12
Glu Phe Cys Met Ser Ile Thr Lys Lys Asp Ala Asn Pro Asn Asn Gly
1               5                   10                  15
Gln Thr Gln Leu Glu Ala Ala Arg Met Glu Leu Thr Asp Leu Ile Asn
            20                  25                  30
Ala Lys Ala Met Thr Leu Ala Ser Leu Gln Asp Tyr Ala Lys Ile Glu
        35                  40                  45
Ala Ser Leu Ser Ser Ala Tyr Ser Glu Ala Glu Thr Val Asn Asn Asn
    50                  55                  60
Leu Asn Ala Thr Leu Glu Gln Leu Lys Met Ala Lys Thr Asn Leu Glu
65                  70                  75                  80
Ser Ala Ile Asn Gln Ala Asn Thr Asp Lys Thr Thr Phe Asp Asn Glu
                85                  90                  95
His Pro Asn Leu Val Glu Ala Tyr Lys Ala Leu Lys Thr Thr Leu Glu
            100                 105                 110
Gln Arg Ala Thr Asn Leu Glu Gly Leu Ser Ser Thr Ala Tyr Asn Gln
        115                 120                 125
Ile Arg Asn Asn Leu Val Asp Leu Tyr Asn Lys Ala Ser Ser Leu Ile
    130                 135                 140
Thr Lys Thr Leu Asp Pro Leu Asn Gly Gly Thr Leu Leu Asp Ser Asn
145                 150                 155                 160
Glu Ile Thr Thr Val Asn Arg Asn Ile Asn Asn Thr Leu Ser Thr Ile
                165                 170                 175
Asn Glu Gln Lys Thr Asn Ala Asp Ala Leu Ser Asn Ser Phe Ile Lys
            180                 185                 190
Lys Val Ile Gln Asn Asn Glu Gln Ser Phe Val Gly Thr Phe Thr Asn
        195                 200                 205
Ala Asn Val Gln Pro Ser Asn Tyr Ser Phe Val Ala Phe Ser Ala Asp
    210                 215                 220
Val Thr Pro Val Asn Tyr Lys Tyr Ala Arg Arg Thr Val Trp Asn Gly
225                 230                 235                 240
Asp Glu Pro Ser Ser Arg Ile Leu Ala Asn Thr Asn Ser Ile Thr Asp
                245                 250                 255
Val Ser Trp Ile Tyr Ser Leu Ala Gly Thr Asn Thr Lys Tyr Gln Phe
            260                 265                 270
Ser Phe Ser Asn Tyr Gly Pro Ser Thr Gly Tyr Leu Tyr Phe Pro Tyr
        275                 280                 285

Lys Leu Val Lys Ala Ala Asp Ala Asn Asn Val Gly Leu Gln Tyr Lys
    290                 295                 300
Leu Asn Asn Gly Asn Val Gln Gln Val Glu Phe Ala Thr Ser Thr Ser
305                 310                 315                 320
Ala Asn Asn Thr Thr Ala Asn Pro Thr Pro Ala Val Asp Glu Ile Lys
                325                 330                 335
Val Ala Lys Ile Val Leu Ser Gly Leu Arg Phe Gly Gln Asn Thr Ile
            340                 345                 350
Glu Leu Ser Val Pro Thr Gly Glu Gly Asn Met Asn Lys Val Ala Pro
        355                 360                 365
Met Ile Gly Asn Ile Tyr Leu Ser Ser Asn Glu Asn Asn Ala Asp Lys
    370                 375                 380
<210> 13
<211> 23
<212> DNA
<213> Artificial Sequence
<220>
<223> Description of Artificial Sequence:Primer 40KG-1
<400> 13
gagttaacag atctaatcaa tgc 23
<210> 14
<211> 22
<212> DNA
<213> Artificial Sequence
<220>
<223> Description of Artificial Sequence::Primer 40KG-2R
<400> 14
gttctaatgt tgcctgcagg tt 22
<210> 15
<211> 23
<212> DNA
<213> Artificial Sequence
<220>
<223> Description of Artificial Sequence:Primer 40KG-2
<400> 15
gttaacaata acctgcaggc aac 23
<210> 16
<211> 21
<212> DNA

<213> Artificial Sequence
<220>
<223> Description of Artificial Sequence:Primer 40KG-3R
<400> 16
gataacgtat tctggatatt c                                                21
<210> 17
<211> 36
<212> DNA
<213> Artificial Sequence
<220>
<223> Description of Artificial Sequence:Primer 40KG-3A
<400> 17
gagattacta cagctaataa gaatatccag aatacg                                36
<210> 18
<211> 34
<212> DNA
<213> Artificial Sequence
<220>
<223> Description of Artificial Sequence:Primer 40KG-4RA
<400> 18
cagcactaaa agcaacaaaa ctgtactggc tagg                                  34
<210> 19
<211> 24
<212> DNA
<213> Artificial Sequence
<220>
<223> Description of Artificial Sequence:Primer 40KG-4
<400> 19
gctaatgttc aacctagcca gtac                                             24
<210> 20
<211> 28
<212> DNA
<213> Artificial Sequence
<220>
<223> Description of Artificial Sequence:Primer 40KG-5R
<400> 20
ggattagctg tagtctgctg tgcgctag                                         28
<210> 21

<211> 27
<212> DNA
<213> Artificial Sequence
<220>
<223> Description of Artificial Sequence:Primer 40KG-5
<400> 21
gccacttcaa ctagcgcaca gcagact                                          27
<210> 22
<211> 37
<212> DNA
<213> Artificial Sequence
<220>
<223> Description of Artificial Sequence:Primer 40KG-6R
<400> 22
gcgtcgactc acttatcagc attattttca tttgagc                               37
<210> 23
<211> 357
<212> PRT
<213> Mycoplasma gallisepticum
<223> Modified TT1-1 portion (downstream of BglI) of
      pNZ40K-S
<400> 23
Thr Asp Leu Ile Asn Ala Lys Ala Met Thr Leu Ala Ser Leu Gln Asp
1               5                   10                  15
Tyr Ala Lys Ile Glu Ala Ser Leu Ser Ser Ala Tyr Ser Glu Ala Glu
            20                  25                  30
Thr Val Asn Asn Asn Leu Gln Ala Thr Leu Glu Gln Leu Lys Met Ala
        35                  40                  45
Lys Thr Asn Leu Glu Ser Ala Ile Asn Gln Ala Asn Thr Asp Lys Thr
    50                  55                  60
Thr Phe Asp Asn Glu His Pro Asn Leu Val Glu Ala Tyr Lys Ala Leu
65                  70                  75                  80
Lys Thr Thr Leu Glu Gln Arg Ala Thr Asn Leu Glu Gly Leu Ser Ser
                85                  90                  95
Thr Ala Tyr Asn Gln Ile Arg Asn Asn Leu Val Asp Leu Tyr Asn Lys
            100                 105                 110
Ala Ser Ser Leu Ile Thr Lys Thr Leu Asp Pro Leu Asn Gly Gly Thr
        115                 120                 125
Leu Leu Asp Ser Asn Glu Ile Thr Thr Ala Asn Lys Asn Ile Gln Asn
    130                 135                 140

Thr Leu Ser Thr Ile Asn Glu Gln Lys Thr Asn Ala Asp Ala Leu Ser
145                 150                 155                 160
Asn Ser Phe Ile Lys Lys Val Ile Gln Asn Asn Glu Gln Ser Phe Val
                165                 170                 175
Gly Thr Phe Thr Asn Ala Asn Val Gln Pro Ser Gln Tyr Ser Phe Val
            180                 185                 190
Ala Phe Ser Ala Asp Val Thr Pro Val Asn Tyr Lys Tyr Ala Arg Arg
        195                 200                 205
Thr Val Trp Asn Gly Asp Glu Pro Ser Ser Arg Ile Leu Ala Asn Thr
    210                 215                 220
Asn Ser Ile Thr Asp Val Ser Trp Ile Tyr Ser Leu Ala Gly Thr Asn
225                 230                 235                 240
Thr Lys Tyr Gln Phe Ser Phe Ser Asn Tyr Gly Pro Ser Thr Gly Tyr
                245                 250                 255
Leu Tyr Phe Pro Tyr Lys Leu Val Lys Ala Ala Asp Ala Asn Asn Val
            260                 265                 270
Gly Leu Gln Tyr Lys Leu Asn Asn Gly Asn Val Gln Gln Val Glu Phe
        275                 280                 285
Ala Thr Ser Thr Ser Ala Gln Gln Thr Thr Ala Asn Pro Thr Pro Ala
    290                 295                 300
Val Asp Glu Ile Lys Val Ala Lys Ile Val Leu Ser Gly Leu Arg Phe
305                 310                 315                 320
Gly Gln Asn Thr Ile Glu Leu Ser Val Pro Thr Gly Glu Gly Asn Met
                325                 330                 335
Asn Lys Val Ala Pro Met Ile Gly Asn Ile Tyr Leu Ser Ser Asn Glu
            340                 345                 350
Asn Asn Ala Asp Lys
        355
<210> 24
<211> 1082
<212> DNA
<213> Mycoplasma gallisepticum
<223> Modified TTM-1 portion (downstream of BglI) of
      pNZ40K-S
<400> 24
acagatctaa tcaatgctaa agcgatgaca ttagcttcac tacaagacta tgccaagatt    60
gaagctagtt tatcatctgc ttatagtgaa gctgaaacag ttaacaataa cctgcaggca   120
acattagaac aactaaaaat ggctaaaact aatttagaat cagccatcaa ccaagctaat   180
acggataaaa cgacttttga taatgaacac ccaaatttag ttgaagcata caaagcacta   240
aaaaccactt tagaacaacg tgctactaac cttgaaggtt tgtcatcaac tgcttataat   300
caaattcgca ataatttagt ggatctatac aataaagcta gtagtttaat aactaaaaca   360

ctagatccac taaatggggg aacgctttta gattctaatg agattactac agctaataag 420
aatatccaga atacgttatc aactattaat gaacaaaaga ctaatgctga tgcattatct 480
aatagtttta ttaaaaaagt gattcaaaat aatgaacaaa gttttgtagg gacttttaca 540
aacgctaatg ttcaacctag ccagtacagt tttgttgctt ttagtgctga tgtaacaccc 600
gtcaattata aatatgcaag aaggaccgtt tggaatggtg atgaaccttc aagtagaatt 660
cttgcaaaca cgaatagtat cacagatgtt tcttggattt atagtttagc tggaacaaac 720
acgaagtacc aatttagttt tagcaactat ggtccatcaa ctggttattt atatttccct 780
tataagttgg ttaaagcagc tgatgctaat aacgttggat tacaatacaa attaaataat 840
ggaaatgttc aacaagttga gtttgccact tcaactagcg cacagcagac tacagctaat 900
ccaactccag cagttgatga gattaaagtt gctaaaatcg ttttatcagg tttaagattt 960
ggccaaaaca caatcgaatt aagtgttcca acgggtgaag gaaatatgaa taaagttgcg 1020
ccaatgattg gcaacattta tcttagctca aatgaaaata atgctgataa gtgagtcgac 1080
gc 1082
<210> 25
<211> 1224
<212> PRT
<213> Mycoplasma gallisepticum
<223> MGC3 encoded by mgc3 gene
<400> 25
Met Asn Ile Ser Lys Lys Leu Lys Ser Tyr Thr Leu Ile Gly Gly Leu
1               5                   10                  15
Ala Val Phe Gly Ala Leu Gly Ser Ala Ser Phe Gly Phe Lys Gln Ser
            20                  25                  30
Asp Lys Ser Asn Asp Asn Thr Gln Leu Val Asn Gln Ala Arg Thr Leu
        35                  40                  45
Asp Ala Asn Ser Val Arg Leu Ala Gly Leu Gly Gln Asn Gly Ser Leu
    50                  55                  60
Phe Asn Thr Val Leu Arg Asp Val Asp Asp Asn Phe Ile Thr Ala Ala
65                  70                  75                  80
Asn Gly Thr Ile Ile Lys Leu Asp Ser Phe Thr Lys Pro Leu Tyr Gly
                85                  90                  95
Leu Asp Leu Ser Asp Asp Cys Gly Gly Tyr Lys Val Lys Gln Ile Val
            100                 105                 110
Ser Asp Tyr Thr Thr Ser Arg Asn Arg Phe Asp Gln Arg Gln Thr Arg
        115                 120                 125
Ala Tyr Tyr Ala Leu Leu Val Asn Asp Glu Ala Asn Val His Leu Lys
    130                 135                 140
Arg Ile Asn Thr Asn Ser Asn Arg Ile Gly Asn Arg Asn Asn Asn Ser
145                 150                 155                 160
Lys Phe Val Ile Gly Gly Val Asp Asn Pro Ala His Val Ile Arg Phe
                165                 170                 175

Thr Asp Asp Gly Thr Lys Phe Asn Phe Thr Asn Gln Thr Gln Gly Glu
            180                 185                 190
Ile Val Asn Asp Phe Ile Leu Asp Ala Pro Ile Leu Pro Lys Asp Leu
        195                 200                 205
His Pro Asp Trp Tyr Asn Leu Tyr Ile Gln Arg Lys Ile Leu Pro Asn
    210                 215                 220
Asp Val Asn Thr Ala Val Val Pro Trp Pro Val Gly Arg Val Ser Gly
225                 230                 235                 240
Thr Asn Ala Asp Asp Gly Met Phe Asp Cys Gly Asn Gly Gln Ile Thr
                245                 250                 255
Asn Thr Asp Pro Ile Ala Gln Thr Lys Thr Thr Thr Asp Asn Gln Asn
            260                 265                 270
Pro Ser Thr Phe Asn Ser Gly Ala Met Pro Gly Ala Asn Asn Arg Tyr
        275                 280                 285
Asp Ser Gln Leu Asn Val Lys His Arg Ile Lys Thr Ser Phe Gln Leu
    290                 295                 300
Asp Glu Arg Ile Asn Thr Asn Ser Asn Arg Ile Gly Asn Arg Asn Asn
305                 310                 315                 320
Asn Ser Lys Phe Val Ile Gly Gly Val Asp Asn Pro Ala His Val Ile
                325                 330                 335
Arg Phe Thr Asp Asp Gly Thr Lys Phe Asn Phe Thr Asn Gln Thr Gln
            340                 345                 350
Gly Glu Ile Val Asn Asp Phe Ile Leu Asp Ala Pro Ile Leu Pro Lys
        355                 360                 365
Asp Leu His Pro Asp Trp Tyr Asn Leu Tyr Ile Gln Arg Lys Ile Leu
    370                 375                 380
Pro Asn Asp Val Asn Thr Ala Val Val Pro Trp Pro Val Gly Arg Val
385                 390                 395                 400
Ser Gly Thr Asn Ala Asp Asp Gly Met Phe Asp Cys Gly Asn Gly Gln
                405                 410                 415
Ile Thr Asn Thr Asp Pro Ile Ala Gln Thr Lys Thr Thr Thr Asp Asn
            420                 425                 430
Gln Asn Pro Ser Thr Phe Asn Ser Gly Ala Met Pro Gly Ala Asn Asn
        435                 440                 445
Arg Tyr Asp Ser Gln Leu Asn Val Lys His Arg Ile Lys Thr Ser Phe
    450                 455                 460
Gln Leu Asp Glu Lys Phe Val Tyr Pro Glu Trp Thr Gly Ser Glu Glu
465                 470                 475                 480
Asn Lys Asn Ile Thr Arg Leu Ala Thr Gly Ser Leu Pro Ser Asn Glu
                485                 490                 495
Arg Tyr Trp Ile Leu Asp Ile Pro Gly Thr Pro Gln Val Thr Leu Lys
            500                 505                 510

Glu Asp Ser Val Asn Val Phe Ser Arg Leu Tyr Leu Asn Ser Val Asn
        515                 520                 525
Ser Leu Ser Phe Ile Gly Asp Ser Ile Tyr Ile Phe Gly Thr Ser Glu
    530                 535                 540
Leu Pro Ser Leu Trp Tyr Tyr Ser Phe Pro Thr Arg Leu Ser Asp Leu
545                 550                 555                 560
Thr Ala Leu Asn Gln Val Lys Thr Asp Asp Ile Glu Ala Ser Ser Thr
                565                 570                 575
Asp Asn Gly Thr Thr Thr Asn Gly Thr Thr Thr Thr Ala Asp Thr Ser
            580                 585                 590
Ser Gly Ser Thr Gly Ala Gly Thr Gly Asn Thr Thr Asn Thr Ser Gln
        595                 600                 605
Thr Val Ser Asn Pro Thr Leu Asn Thr Tyr Arg Ser Phe Gly Ile Asp
    610                 615                 620
Ser Lys Pro Thr Ser Ala Asn Lys Ile Asp Glu Thr Asn Trp Ala Asp
625                 630                 635                 640
Pro Asn Val Ile Glu Ala Arg Ile Tyr Ala Glu Tyr Arg Leu Gly Ile
                645                 650                 655
Gln Asn Glu Ile Pro Ile Thr Asn Ala Gly Asn Phe Ile Arg Asn Thr
            660                 665                 670
Ile Gly Gly Val Gly Phe Thr Ser Thr Gly Ser Arg Val Val Leu Arg
        675                 680                 685
Ala Ser Tyr Asn Gly Asp Gln Arg Pro Thr Gly Asn Phe Gln Pro Phe
    690                 695                 700
Leu Tyr Val Phe Gly Tyr Leu Gly Tyr Gln Gln Thr Arg Thr Gly Thr
705                 710                 715                 720
Phe Trp Tyr Gly Thr Tyr Lys Leu Leu Asn Asn Ser Pro Tyr Asp Val
                725                 730                 735
Leu Asp Ser Pro Arg Val Gly Thr Glu Thr Asn Gln Phe Arg Arg Thr
            740                 745                 750
Ser Leu Thr Tyr Pro Val Met Gly Gly Tyr Leu Thr Glu Glu Gly Ala
        755                 760                 765
Arg Ser Phe Ser Asn Thr Pro Tyr Ile Arg Ala Gln Gly Asp Thr Pro
    770                 775                 780
Glu Ser Arg Ser Ile Phe Gln Ser Gly Tyr Ser Asp Asn Thr Tyr Glu
785                 790                 795                 800
Tyr Ile Gln Ser Val Leu Gly Phe Asp Gly Ile Arg Asn Asn Leu Asn
                805                 810                 815
Val Gly Val Lys Ala Ser Ser Phe Leu Asn Ser Asn Arg Pro Asn Pro
            820                 825                 830
Asn Gly Leu Glu Met Ile Ala Ala Thr Thr Tyr Leu Arg Ser Gln Ile
        835                 840                 845

Gly Leu Ala Arg Thr Ser Gly Leu Pro Asn Gln Gln Pro Phe Gly Thr
    850                 855                 860
Thr His Gln Val Ile Ser Val Ser Pro Gly Asp Gln Phe Ser Ser Ile
865                 870                 875                 880
Lys Asn Ile Arg Thr Ile Phe Pro Gly Asn Gln Leu Trp Tyr Phe Leu
                885                 890                 895
Phe Thr Asn Glu Asn Asn Lys Ser Ser Val Tyr Thr Leu Arg Leu Ala
            900                 905                 910
Asp Ser Ser Asn Pro Asp Ala Ser Ser Ser Phe Ser Pro Thr Ser Leu
        915                 920                 925
Ile Asp Val Asn Glu Ile Gly Val Ile Leu Pro Leu Leu Asp Asn Ser
    930                 935                 940
Phe Tyr Thr Val Asn Ala Ala Gly Asn Val Ala Leu Phe Ser Ser Asn
945                 950                 955                 960
Pro Gly Ser Pro Gly Ser Tyr Thr Ala Val Asn Thr Phe Asn Gln Asn
                965                 970                 975
Leu Ser Asp Ile Ala Phe Glu Gly Ser Gly Ala Lys Tyr Thr Ser Asp
            980                 985                 990
Phe Trp Gly Thr Ile Gln Phe Lys Pro Asp Glu Tyr Leu Ile Gln Asn
        995                 1000                1005
Gly Phe Thr Ser Gln Val Ala Arg Asn Phe Val Thr Asn Gln Ser Phe
    1010                1015                1020
Leu Asn Ser Leu Val Asp Phe Thr Pro Ala Asn Ala Gly Thr Asn Tyr
1025                1030                1035                1040
Arg Val Val Val Asp Pro Asp Gly Asn Leu Thr Asn Gln Asn Leu Pro
                1045                1050                1055
Leu Lys Val Gln Ile Gln Tyr Leu Asp Gly Lys Tyr Tyr Asp Ala Lys
            1060                1065                1070
Leu Lys Asn Asn Asn Leu Val Thr Phe Ser Tyr Asn Asn Phe Gly Ala
        1075                1080                1085
Leu Pro Ser Trp Val Val Pro Thr Ala Ile Gly Ser Thr Leu Gly Ile
    1090                1095                1100
Leu Ala Ile Met Ile Ile Leu Gly Leu Ala Ile Gly Ile Pro Leu Arg
1105                1110                1115                1120
Ala Gln Arg Lys Leu Gln Asp Lys Gly Phe Lys Thr Thr Phe Lys Lys
                1125                1130                1135
Val Asp Thr Leu Thr Ala Ala Val Gly Ser Val Tyr Lys Lys Ile Ile
            1140                1145                1150
Thr Gln Thr Ala Asn Val Lys Lys Lys Pro Ala Ala Leu Gly Ala Gly
        1155                1160                1165
Lys Ser Gly Asp Lys Lys Pro Ala Ala Ala Ala Lys Pro Ala Ala Pro
    1170                1175                1180

Ala Lys Pro Ser Ala Pro Lys Ala Ser Ser Pro Ala Lys Pro Thr Gly
1185                1190                1195                1200
Pro Lys Ser Gly Ala Pro Thr Lys Pro Thr Ala Pro Lys Pro Ala Ala
                1205                1210                1215
Pro Lys Pro Thr Ala Pro Lys Glu
            1220
<210> 26
<211> 26
<212> DNA
<213> Artificial Sequence
<220>
<223> Description of Artificial Sequence:Primer M11-B
<400> 26
gcgaattcgg ttctgcaagc tttggc                                          26
<210> 27
<211> 20
<212> DNA
<213> Artificial Sequence
<220>
<223> Description of Artificial Sequence:Primer M11-2R
<400> 27
aacaacgaac cctgttgtcc                                                 20
<210> 28
<211> 20
<212> DNA
<213> Artificial Sequence
<220>
<223> Description of Artificial Sequence:Primer M11-2
<400> 28
gcaggtcttg gacaacaggg                                                 20
<210> 29
<211> 20
<212> DNA
<213> Artificial Sequence
<220>
<223> Description of Artificial Sequence:Primer M11-3R
<400> 29
ttgataattg ttccctgagc                                                 20
<210> 30

<211> 20
<212> DNA
<213> Artificial Sequence
<220>
<223> Description of Artificial Sequence:Primer M11-3
<400> 30
tttataacag cagctcaggg                                                    20
<210> 31
<211> 30
<212> DNA
<213> Artificial Sequence
<220>
<223> Description of Artificial Sequence:Primer M11-4RB
<400> 31
caccaattac aaacttagaa ttttggtttc                                         30
<210> 32
<211> 30
<212> DNA
<213> Artificial Sequence
<220>
<223> Description of Artificial Sequence:Primer M11-4B
<400> 32
agaattggta atagaaacca aaattctaag                                         30
<210> 33
<211> 20
<212> DNA
<213> Artificial Sequence
<220>
<223> Description of Artificial Sequence:Primer M11-5R
<400> 33
tgagtttgct gtgtaaactg                                                    20
<210> 34
<211> 30
<212> DNA
<213> Artificial Sequence
<220>
<223> Description of Artificial Sequence:Primer M11-5A
<400> 34

taaatttcag tttacacagc aaactcaagg 30
<210> 35
<211> 30
<212> DNA
<213> Artificial Sequence
<220>
<223> Description of Artificial Sequence:Primer M11-7RA
<400> 35
agtagctaat cttgtaatct gtttattctc 30
<210> 36
<211> 20
<212> DNA
<213> Artificial Sequence
<220>
<223> Description of Artificial Sequence:Primer M11-7
<400> 36
gttctgaaga gaataaacag 20
<210> 37
<211> 20
<212> DNA
<213> Artificial Sequence
<220>
<223> Description of Artificial Sequence:Primer M11-KR
<400> 37
tggtaattca gaggtaccaa 20
<210> 38
<211> 20
<212> DNA
<213> Artificial Sequence
<220>
<223> Description of Artificial Sequence:Primer M11-K
<400> 38
ttatattttt ggtacctctg 20
<210> 39
<211> 30
<212> DNA
<213> Artificial Sequence
<220>

<223> Description of Artificial Sequence:Primer M11-8R
<400> 39
tgtcgttgtt ccctgtgttg ttgtaccctg                                        30
<210> 40
<211> 30
<212> DNA
<213> Artificial Sequence
<220>
<223> Description of Artificial Sequence:Primer M11-8
<400> 40
tcaagcactg atcagggtac aacaacacag                                        30
<210> 41
<211> 25
<212> DNA
<213> Artificial Sequence
<220>
<223> Description of Artificial Sequence:Primer M11-10R
<400> 41
ctgtttgaga agtctgagta gtctg                                             25
<210> 42
<211> 25
<212> DNA
<213> Artificial Sequence
<220>
<223> Description of Artificial Sequence:Primer M11-10
<400> 42
tgctggaaca ggacagacta ctcag                                             25
<210> 43
<211> 30
<212> DNA
<213> Artificial Sequence
<220>
<223> Description of Artificial Sequence:Primer M11-12RA
<400> 43
cgtcgtaagg gctgttctgt aaaagcttat                                        30
<210> 44
<211> 30
<212> DNA

<213> Artificial Sequence
<220>
<223> Description of Artificial Sequence:Primer M11-12A
<400> 44
cggaacatat aagcttttac agaacagccc                                        30
<210> 45
<211> 20
<212> DNA
<213> Artificial Sequence
<220>
<223> Description of Artificial Sequence:Primer M11-XR
<400> 45
cagcaatcat ttctagaccg                                                   20
<210> 46
<211> 30
<212> DNA
<213> Artificial Sequence
<220>
<223> Description of Artificial Sequence:Primer M11-XA
<400> 46
aaatccaaac ggtctagaaa tgattgctgc                                        30
<210> 47
<211> 30
<212> DNA
<213> Artificial Sequence
<220>
<223> Description of Artificial Sequence:Primer M11-13RA
<400> 47
taatgtataa acactagatt tctgattttc                                        30
<210> 48
<211> 30
<212> DNA
<213> Artificial Sequence
<220>
<223> Description of Artificial Sequence:Primer M11-13A
<400> 48
tattcacaaa tgaaaatcag aaatctagtg                                        30
<210> 49

<211> 30
<212> DNA
<213> Artificial Sequence
<220>
<223> Description of Artificial Sequence:Primer M11-14RA
<400> 49
accttcaaaa gcaatatcag acagctgctg                                        30
<210> 50
<211> 30
<212> DNA
<213> Artificial Sequence
<220>
<223> Description of Artificial Sequence:Primer M11-14A
<400> 50
tacatttaat cagcagctgt ctgatattgc                                        30
<210> 51
<211> 30
<212> DNA
<213> Artificial Sequence
<220>
<223> Description of Artificial Sequence:Primer M11-15RA
<400> 51
ctgtttaaga agctttgctg tgtaacgaag                                        30
<210> 52
<211> 30
<212> DNA
<213> Artificial Sequence
<220>
<223> Description of Artificial Sequence:Primer M11-15A
<400> 52
gctagaaact tcgttacaca gcaaagcttc                                        30
<210> 53
<211> 30
<212> DNA
<213> Artificial Sequence
<220>
<223> Description of Artificial Sequence:Primer M11-16RA
<400> 53

taggttttgg tttgtcagct gaccatcagg                                    30
<210> 54
<211> 30
<212> DNA
<213> Artificial Sequence
<220>
<223> Description of Artificial Sequence:Primer M11-16A
<400> 54
tggttgatcc tgatggtcag ctgacaaacc                                    30
<210> 55
<211> 30
<212> DNA
<213> Artificial Sequence
<220>
<223> Description of Artificial Sequence:Primer M11-GTR
<400> 55
gcgtcgactt attctttggg agcggttggt                                    30
<210> 56
<211> 36
<212> DNA
<213> Artificial Sequence
<220>
<223> Description of Artificial Sequence:Linker
      H'-S-H-S-P-S1
<400> 56
agctggccgg gggggccaag cttgcatgcc tgcagg                             36
<210> 57
<211> 36
<212> DNA
<213> Artificial Sequence
<220>
<223> Description of Artificial Sequence:Linker
      H'-S-H-S-P-S2
<400> 57
agctggacgt ccgtacgttc gaaccggggg ggccgg                             36
<210> 58
<211> 35
<212> DNA

<213> Artificial Sequence
<220>
<223> Description of Artificial Sequence:Linker S-B-E1
<400> 58
tcgacatttt tatgtaccga gctgccgggg gggcg                                35
<210> 59
<211> 35
<212> DNA
<213> Artificial Sequence
<220>
<223> Description of Artificial Sequence:Linker S-B-E2
<400> 59
ttaagcgggg gggccgtcga gccatgtatt tttac                                35
<210> 60
<211> 30
<212> DNA
<213> Artificial Sequence
<220>
<223> Description of Artificial Sequence:Primer M11-Sfi
<400> 60
gaagatggaa ttcggccggg ggggccaagc                                      30
<210> 61
<211> 30
<212> DNA
<213> Artificial Sequence
<220>
<223> Description of Artificial Sequence::Primer M11-5RB
<400> 61
cattaacaat ttcaccttga gtttgctttg                                      30
<210> 62
<211> 30
<212> DNA
<213> Artificial Sequence
<220>
<223> Description of Artificial Sequence:Primer M11-5C
<400> 62
atgatgggac taaatttcag tttacaaagc                                      30
<210> 63

<211> 30
<212> DNA
<213> Artificial Sequence
<220>
<223> Description of Artificial Sequence:Primer M11-KRA
<400> 63
gtaattcaga ggtaccaaaa atataaatac                                    30
<210> 64
<211> 30
<212> DNA
<213> Artificial Sequence
<220>
<223> Description of Artificial Sequence:Primer pCMV-1
<400> 64
gggctgcaga gttattaata gtaatcaatt                                    30
<210> 65
<211> 30
<212> DNA
<213> Artificial Sequence
<220>
<223> Description of Artificial Sequence:Primer pPec1R
<400> 65
gccaggcgcg ccatttaccg tcattgacgt                                    30
<210> 66
<211> 30
<212> DNA
<213> Artificial Sequence
<220>
<223> Description of Artificial Sequence:Primer pCMV-o1
<400> 66
acgtcaatga cggtaaatgg cgcgcctggc                                    30
<210> 67
<211> 30
<212> DNA
<213> Artificial Sequence
<220>
<223> Description of Artificial Sequence:Primer pCMV-R1
<400> 67

cgtctagagg atctgacggt tcactaaacc                                    30
<210> 68
<211> 29
<212> DNA
<213> Artificial Sequence
<220>
<223> Description of Artificial Sequence:Primer pGHP-F
<400> 68
gccaagcttg gccttattgg cctgcaggt                                     29
<210> 69
<211> 30
<212> DNA
<213> Artificial Sequence
<220>
<223> Description of Artificial Sequence:Primer pGHP-R
<400> 69
acgaattcgc caataaggca taaacacaaa                                    30
<210> 70
<211> 23
<212> DNA
<213> Artificial Sequence
<220>
<223> Description of Artificial Sequence:Linker 1
<400> 70
tcgacgatat cgccttattg gcg                                           23
<210> 71
<211> 23
<212> DNA
<213> Artificial Sequence
<220>
<223> Description of Artificial Sequence:Linker 2
<400> 71
aattcgccaa taaggcgata tcg                                           23
<210> 72
<211> 16
<212> DNA
<213> Artificial Sequence
<220>

<223> Description of Artificial Sequence:Linker 3
<400> 72
agctccacgc ctctag                                                    16
<210> 73
<211> 12
<212> DNA
<213> Artificial Sequence
<220>
<223> Description of Artificial Sequence:Linker 4
<400> 73
ctagaggcgt gg                                                        12
<210> 74
<211> 30
<212> DNA
<213> Artificial Sequence
<220>
<223> Description of Artificial Sequence:Primer
      PolyA-SfiF2
<400> 74
cttggcctta ttggcctaag atacattgat                                     30
<210> 75
<211> 30
<212> DNA
<213> Artificial Sequence
<220>
<223> Description of Artificial Sequence:Primer
      PolyA-SalKpn
<400> 75
tgtggtaccg tcgacgattc acagtcccaa                                     30
<210> 76
<211> 22
<212> DNA
<213> Artificial Sequence
<220>
<223> Description of Artificial Sequence:Primer 40KS-B
<400> 76
gcactcggat ccaatcatgc ac                                             22
<210> 77

<211> 30
<212> DNA
<213> Artificial Sequence
<220>
<223> Description of Artificial Sequence:Primer pMG40K-1
<400> 77
ggggatcctt ctatgaataa gaaaagaatc                                        30
<210> 78
<211> 1042
<212> PRT
<213> Mycoplasma gallisepticum
<223> Modified MGC3 antigen (M11-BTR)
<400> 78
Glu Phe Gly Ser Ala Ser Phe Gly Phe Lys Gln Ser Asp Lys Ser Asn
1               5                   10                  15
Asp Asn Thr Gln Leu Val Asn Gln Ala Arg Thr Leu Asp Ala Asn Ser
            20                  25                  30
Val Arg Leu Ala Gly Leu Gly Gln Gln Gly Ser Leu Phe Asn Thr Val
        35                  40                  45
Leu Arg Asp Val Asp Asp Asn Phe Ile Thr Ala Ala Gln Gly Thr Ile
    50                  55                  60
Ile Lys Leu Asp Ser Phe Thr Lys Pro Leu Tyr Gly Leu Asp Leu Ser
65                  70                  75                  80
Asp Asp Phe Ala Gly Tyr Lys Val Lys Gln Ile Val Ser Asp Tyr Thr
                85                  90                  95
Thr Ser Arg Asn Arg Phe Asp Gln Arg Gln Thr Arg Ala Tyr Tyr Ala
            100                 105                 110
Leu Leu Val Asn Asp Glu Ala Asn Val His Leu Lys Arg Ile Asn Thr
        115                 120                 125
Asn Ser Asn Arg Ile Gly Asn Arg Asn Gln Asn Ser Lys Phe Val Ile
    130                 135                 140
Gly Gly Val Asp Asn Pro Ala His Val Ile Arg Phe Thr Asp Asp Gly
145                 150                 155                 160
Thr Lys Phe Gln Phe Thr Gln Gln Thr Gln Gly Glu Ile Val Asn Asp
                165                 170                 175
Phe Ile Leu Asp Ala Pro Ile Leu Pro Lys Asp Leu His Pro Asp Trp
            180                 185                 190
Tyr Asn Leu Tyr Ile Gln Arg Lys Ile Leu Pro Asn Asp Val Asn Thr
        195                 200                 205
Ala Val Val Pro Trp Pro Val Gly Arg Val Ser Gly Thr Asn Ala Asp
    210                 215                 220

Asp Gly Met Phe Asp Phe Gly Asn Gly Gln Ile Thr Asn Thr Asp Pro
225                 230                 235                 240
Ile Ala Gln Thr Lys Thr Thr Thr Asp Asn Gln Asn Pro Ser Thr Phe
                245                 250                 255
Asn Ser Gly Ala Met Pro Gly Ala Asn Asn Arg Tyr Asp Ser Gln Leu
            260                 265                 270
Asn Val Lys His Arg Ile Lys Thr Ser Phe Gln Leu Asp Glu Lys Phe
        275                 280                 285
Val Tyr Pro Glu Trp Thr Gly Ser Glu Glu Asn Lys Gln Ile Thr Arg
    290                 295                 300
Leu Ala Thr Gly Ser Leu Pro Ser Asn Glu Arg Tyr Trp Ile Leu Asp
305                 310                 315                 320
Ile Pro Gly Thr Pro Gln Val Thr Leu Lys Glu Asp Ser Val Asn Val
                325                 330                 335
Phe Ser Arg Leu Tyr Leu Asn Ser Val Asn Ser Leu Ser Phe Ile Gly
            340                 345                 350
Asp Ser Ile Tyr Ile Phe Gly Thr Ser Glu Leu Pro Ser Leu Trp Tyr
        355                 360                 365
Tyr Ser Phe Pro Thr Arg Leu Ser Asp Leu Thr Ala Leu Asn Gln Val
    370                 375                 380
Lys Thr Asp Asp Ile Glu Ala Ser Ser Thr Asp Gln Gly Thr Thr Thr
385                 390                 395                 400
Gln Gly Thr Thr Thr Thr Thr Asp Thr Ser Ser Gly Ser Thr Gly Ala
                405                 410                 415
Gly Thr Gly Gln Thr Thr Gln Thr Ser Gln Thr Val Ser Asn Pro Thr
            420                 425                 430
Leu Asn Thr Tyr Arg Ser Phe Gly Ile Asp Ser Lys Pro Thr Ser Ala
        435                 440                 445
Asn Lys Ile Asp Glu Thr Asn Trp Ala Asp Pro Asn Val Ile Glu Ala
    450                 455                 460
Arg Ile Tyr Ala Glu Tyr Arg Leu Gly Ile Gln Asn Glu Ile Pro Ile
465                 470                 475                 480
Thr Asn Ala Gly Asn Phe Ile Arg Asn Thr Ile Gly Gly Val Gly Phe
                485                 490                 495
Thr Ser Thr Gly Ser Arg Val Val Leu Arg Ala Ser Tyr Asn Gly Asp
            500                 505                 510
Gln Arg Pro Thr Gly Asn Phe Gln Pro Phe Leu Tyr Val Phe Gly Tyr
        515                 520                 525
Leu Gly Tyr Gln Gln Thr Arg Thr Gly Thr Phe Trp Tyr Gly Thr Tyr
    530                 535                 540
Lys Leu Leu Gln Asn Ser Pro Tyr Asp Val Leu Asp Ala Ala Arg Val
545                 550                 555                 560

Gly Thr Glu Thr Asn Gln Phe Arg Arg Thr Ser Leu Thr Tyr Pro Val
                565                 570                 575
Met Gly Gly Tyr Leu Thr Glu Glu Gly Ala Arg Ser Phe Ser Asn Thr
            580                 585                 590
Pro Tyr Ile Arg Ala Gln Gly Asp Thr Pro Glu Ser Arg Ser Ile Phe
        595                 600                 605
Gln Ser Gly Tyr Ser Asp Asn Thr Tyr Glu Tyr Ile Gln Ser Val Leu
    610                 615                 620
Gly Phe Asp Gly Ile Arg Asn Asn Leu Asn Val Gly Val Lys Ala Ser
625                 630                 635                 640
Ser Phe Leu Asn Ser Asn Arg Pro Asn Pro Asn Gly Leu Glu Met Ile
                645                 650                 655
Ala Ala Thr Thr Tyr Leu Arg Ser Gln Ile Gly Leu Ala Arg Thr Ser
            660                 665                 670
Gly Leu Pro Asn Gln Gln Pro Phe Gly Thr Thr His Gln Val Ile Ser
        675                 680                 685
Val Ser Pro Gly Asp Gln Phe Ser Ser Ile Lys Asn Ile Arg Thr Ile
    690                 695                 700
Phe Pro Gly Asn Gln Leu Trp Tyr Phe Leu Phe Thr Asn Glu Asn Gln
705                 710                 715                 720
Lys Ser Ser Val Tyr Thr Leu Arg Leu Ala Asp Ser Ser Asn Pro Asp
                725                 730                 735
Ala Ser Ser Ser Phe Ser Pro Thr Ser Leu Ile Asp Val Asn Glu Ile
            740                 745                 750
Gly Val Ile Leu Pro Leu Leu Asp Asn Ser Phe Tyr Thr Val Asn Ala
        755                 760                 765
Ala Gly Asn Val Ala Leu Phe Ser Ser Asn Pro Gly Ser Pro Gly Ser
    770                 775                 780
Tyr Thr Ala Val Asn Thr Phe Asn Gln Gln Leu Ser Asp Ile Ala Phe
785                 790                 795                 800
Glu Gly Ser Gly Ala Lys Tyr Thr Ser Asp Phe Trp Gly Thr Ile Gln
                805                 810                 815
Phe Lys Pro Asp Glu Tyr Leu Ile Gln Asn Gly Phe Thr Ser Gln Val
            820                 825                 830
Ala Arg Asn Phe Val Thr Gln Gln Ser Phe Leu Asn Ser Leu Val Asp
        835                 840                 845
Phe Thr Pro Ala Asn Ala Gly Thr Asn Tyr Arg Val Val Val Asp Pro
    850                 855                 860
Asp Gly Gln Leu Thr Asn Gln Asn Leu Pro Leu Lys Val Gln Ile Gln
865                 870                 875                 880
Tyr Leu Asp Gly Lys Tyr Tyr Asp Ala Lys Leu Lys Asn Asn Asn Leu
                885                 890                 895

Val Thr Phe Ser Tyr Asn Asn Phe Gly Ala Leu Pro Ser Trp Val Val
            900                 905                 910
Pro Thr Ala Ile Gly Ser Thr Leu Gly Ile Leu Ala Ile Met Ile Ile
        915                 920                 925
Leu Gly Leu Ala Ile Gly Ile Pro Leu Arg Ala Gln Arg Lys Leu Gln
    930                 935                 940
Asp Lys Gly Phe Lys Thr Thr Phe Lys Lys Val Asp Thr Leu Thr Ala
945                 950                 955                 960
Ala Val Gly Ser Val Tyr Lys Lys Ile Ile Thr Gln Thr Ala Asn Val
                965                 970                 975
Lys Lys Lys Pro Ala Ala Leu Gly Ala Gly Lys Ser Gly Asp Lys Lys
            980                 985                 990
Pro Ala Ala Ala Ala Lys Pro Ala Ala Pro Ala Lys Pro Ser Ala Pro
        995                 1000                1005
Lys Ala Ser Ser Pro Ala Lys Pro Thr Ala Pro Lys Ser Gly Ala Pro
    1010                1015                1020
Thr Lys Pro Thr Ala Pro Lys Pro Ala Ala Pro Lys Pro Thr Ala Pro
1025                1030                1035                1040
Lys Glu
<210> 79
<211> 3129
<212> DNA
<213> Mycoplasma gallisepticum
<223> Modified mgc3 gene
<400> 79
gaattcggtt ctgcaagctt tggctttaag caatcagata agagtaacga taacacgcaa    60
ttagttaatc aagcaagaac gctagatgct aattctgtta gacttgcagg tcttggacaa   120
cagggttcgt tgttcaatac agttcttaga gatgttgatg ataactttat aacagcagct   180
cagggaacaa ttatcaaatt agatagtttt actaaaccat tatatggttt agatctaagt   240
gatgattttg ctggatacaa agtaaaacaa atagtttcag attacacaac tagcagaaat   300
agatttgatc aaagacaaac aagagcatat tatgctctgt tggttaatga tgaagctaac   360
gttcatttaa aaagaattaa tactaactca aatagaattg gtaatagaaa ccaaaattct   420
aagtttgtaa ttggtggtgt tgataatcca gctcacgtaa ttagatttac tgatgatggg   480
actaaatttc agtttacaca gcaaactcaa ggtgaaattg ttaatgactt cattttagat   540
gcgccaatct tacctaaaga tttacaccca gattggtata acttatacat tcaaagaaag   600
atcttaccaa atgacgtcaa cactgcagtt gttccttggc cagtaggtag agttagtgga   660
acaaatgctg atgatgggat gtttgatttt gggaatggtc aaataactaa tacagatcct   720
attgctcaaa ctaaaaccac tactgataat caaaatcctt caacttttaa ttcaggagca   780
atgcctggtg caaacaatag atacgattct caattgaatg tcaagcatag aattaaaaca   840
tctttccaat tagatgaaaa atttgtttat ccagaatgga ctggttctga agagaataaa   900
cagattacaa gattagctac tggaagtttg ccaagcaacg aaagatattg gattcttgac   960

atacccggga ctccacaagt tactttaaaa gaagattcag ttaacgtatt ttcaagacta 1020
tacttaaact cagttaattc tttatcattc attggtgata gtatttatat ttttggtacc 1080
tctgaattac catcattatg gtactattca ttcccaacta gattatctga tctaaccgct 1140
ttgaatcaag ttaaaacaga tgatattgaa gcttcaagca ctgatcaggg tacaacaaca 1200
cagggaacaa cgacaacaac tgatacatct agtggttcaa caggtgctgg aacaggacag 1260
actactcaga cttctcaaac agtttctaat cctactttaa atacttatcg tagttttgga 1320
attgatagta aaccaacttc tgcaaacaaa atagatgaaa ctaattgggc agatcctaac 1380
gttattgaag caagaatata tgctgaatac agattaggta ttcaaaatga aattccaata 1440
actaatgcag gaaactttat ccgaaacaca attggtggtg ttggttttac ttcaacaggt 1500
tcaagagtag ttttaagagc ttcttataac ggtgatcaac gtccaactgg aaacttccaa 1560
cctttcttat acgtatttgg ttatttagga taccaacaaa ctagaacagg aactttctgg 1620
tacggaacat ataagctttt acagaacagc ccttacgacg tattagatgc tgcaagagta 1680
ggtactgaaa ccaatcaatt tagaagaact tcattaacat accctgttat gggtggatat 1740
ctaactgaag aaggtgctag aagtttctct aatactccat atataagagc acaaggtgac 1800
acaccagaaa gccgaagcat cttccaatct ggctattctg ataatactta tgagtacatt 1860
caatcagttt taggatttga tggaattaga aataacttaa atgttggggt taaagcatca 1920
agcttcttaa actcaaatag accaaatcca aacggtctag aaatgattgc tgcaacaaca 1980
tacttaagat cacaaattgg attagctaga acatctggat taccaaacca acaaccattc 2040
ggaacaactc accaagttat ttcagtatca cctggtgatc agttctcatc aattaagaat 2100
attagaacaa tcttccctgg taaccagtta tggtacttct tattcacaaa tgaaaatcag 2160
aaatctagtg tttatacatt aagattagct gactcaagta accctgatgc gtcaagctca 2220
ttcagtccaa caagtttaat tgacgttaat gaaattggtg taatcttacc tttattagac 2280
aattcattct atacagtaaa tgctgctggt aatgttgcat tgttctcatc aaaccctggt 2340
tctcctggat catatactgc tgtaaataca tttaatcagc agctgtctga tattgctttt 2400
gaaggttctg gtgctaagta tacatctgat ttctggggaa caatccaatt caaacccgat 2460
gagtacttaa ttcaaaatgg gttcactagt caagtggcta gaaacttcgt tacacagcaa 2520
agcttcttaa acagtttagt tgacttcact cctgctaatg ctggtactaa ctaccgtgta 2580
gtggttgatc ctgatggtca gctgacaaac caaaacctac ctctaaaagt tcagatccaa 2640
tacttagatg gtaagtatta tgatgctaaa ttaaagaaca ataatttagt aacattctct 2700
tataacaact ttggcgcctt accttcatgg gtagtgccta cagcaattgg tagtacatta 2760
ggtattcttg caattatgat catcttagga ttagctatcg gtattccttt aagagctcaa 2820
agaaaattac aagacaaagg gttcaaaaca acattcaaaa aagttgatac cttgactgct 2880
gctgttggtt cagtttacaa gaagattatt acccaaactg ctaacgttaa gaaaaaacct 2940
gctgctttag gtgctggtaa atctggtgat aagaaacctg ctgctgctgc taaacctgct 3000
gctccagcta aaccatctgc accaaaagct agctcaccag ctaaaccaac tgcgcctaaa 3060
tctggtgcgc ctacaaaacc aactgctcct aagccagctg ctccaaaacc aaccgctccc 3120
aaagaataa 3129